# EUROPEAN PATENT SPECIFICATION

(11) **EP 2 611 927 B1**
(45) Date of publication and mention of the grant of the patent: **01.08.2018**
(21) Application number: 11822416.1
(22) Date of filing: 29.08.2011
(51) Int. Cl.: C12N 15/113, C12N 15/87, A61K 47/64

(54) **NOVEL SINGLE CHEMICAL ENTITIES AND METHODS FOR DELIVERY OF OLIGONUCLEOTIDES**
NEUE EINZELNE CHEMISCHE EINHEITEN SOWIE VERFAHREN ZUR ABGABE VON OLIGONUKLEOTIDEN
NOUVELLES ENTITÉS CHIMIQUES SIMPLES ET PROCÉDÉS POUR L'ADMINISTRATION D'OLIGONUCLÉOTIDES

(30) Priority: 31.08.2010 US 378609 P
(43) Date of publication of application: 10.07.2013
(73) Proprietor: Sirna Therapeutics, Inc., 300 Third Street Cambridge MA 02142 (US)
(72) Inventor: COLLETTI, Steven, L., West Point Pennsylvania 19486 (US); GOSSELIN, Francis, San Mateo California 94402 (US); JADHAV, Vasant, R., West Point Pennsylvania 19486 (US); SHAW, Anthony, W., West Point Pennsylvania 19486 (US); TELLERS, David, M., West Point Pennsylvania 19486 (US); TUCKER, Thomas, J., West Point Pennsylvania 19486 (US); YUAN, Yu, West Point Pennsylvania 19486 (US); ZEWGE, Daniel, Rahway New Jersey 07065-0907 (US)
(74) Representative: Müller-Boré & Partner Patentanwälte PartG mbB
(86) International application number: PCT/US2011/049479
(87) International publication number: WO 2012/030683

(56) References cited:
- WO-A2-2007/069068
- WO-A2-2008/109105
- WO-A2-2010/039548
- GAGLIONE M ET AL: "Recent progress in chemically modified siRNAs", MINI REVIEWS IN MEDICINAL CHEMISTRY, BENTHAM SCIENCE PUBL, NL, vol. 10, no. 7, 1 June 2010 (2010-06-01), pages 578-595, XP008146246, ISSN: 1389-5575, DOI: 10.2174/138955710791384036

## Description

The present invention relates to modular compositions comprising an siRNA and one or more peptides that are attached to the siRNA via a linker.

Scientific efforts focused on the delivery of oligonucleotides systemically for therapeutic purposes are ongoing. Three highlighted approches to oligonucleotide delivery include 1) lipid nanoparticle (LNP) encapsulation, 2) polymer conjugation and 3) single chemical conjugation. Single chemical conjugation typically employs a targeting ligand or a lipid or a solubilizing group or an endosomolytic peptide or a cell penetrating peptide and/or a combination of two or all four attached to an oligonucleotide. Linkers may be present in the conjugate as well as other functionalities. Single chemical conjugates are known and attachment of the oligonucleotide occurs either at the 5'- or 3'-end of the oligonucleotide, at both ends, or internally. See WO2005/041859; WO2008/036825, WO2009/126933, US2010/0076056 and WO2010/039548.

In this context, Gaglione and Messere (Gaglione, M. and Messere, A.; Mini-Reviews in Medicinal Chemistry, 10; 2010; pp. 578-595) describes chemically modified siRNAs with improved application profiles. Further, WO 2007/069068 A2 describes cell penetrating peptide-nucleic acid conjugates and uses thereof. Furthermore, WO 2008/109105 A2 describes chemically modified, linked double-stranded RNA compositions and uses thereof.

Considerable amount of literature evidence supports the hypothesis that the major hurdles for oligonucleotide delivery are cell uptake and endosomal escape. To improve delivery efficiency, uptake-promoting peptides and/or endosomolytic peptides and/or charge shielding groups may be required in very condensed topology. In this regard, multi-functional platforms and internal modifications offer unique opportunities to fulfill this requirement.
The present invention relates to the following items:
1. A modular composition comprising
   1) an siRNA;
   2) one or more linkers according to the following formula: 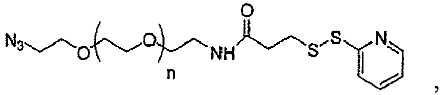 wherein n = 9,
      wherein the linkers are attached to the guide strand of the siRNA at the 2'-position of the ribose rings excluding the terminal 3'- and/or 5'-positions of the guide strand, or to the passenger strand of the siRNA at the 2'-position of the ribose rings excluding the terminal 3'- and/or 5'-positions of the passenger strand; and
   3) one or more peptides according to SEQ ID NO: 19, wherein the peptides are attached to the linkers.
2. The modular composition according to item 1 which further comprises cholesterol, wherein cholesterol is attached to the siRNA at the 2'-position of the ribose rings or the 3'-position of the siRNA.
3. The modular composition according to item 1 which further comprises cholesterol, wherein cholesterol is attached to the siRNA at the 2'-position of the ribose rings and/or the terminal 3'- and/or 5'-positions of the siRNA.
The figures show:
FIG. 1 SSB mRNA levels in HeLa cells treated with compound C4-1.
FIG. 2 SSB mRNA levels in HeLa cells treated with compound C4-5.
FIG. 3 SSB mRNA levels in HeLa cells treated with compound C4-8.
FIG. 4 SSB mRNA levels in HeLa cells treated with compound C4-10.
FIG. 5 SSB mRNA levels in HeLa cells treated with compound C6-1.
FIG. 6 SSB mRNA levels in HeLa cells treated with compound C6-2.
FIG. 7 SSB mRNA levels in HeLa cells treated with compound C7-1.
FIG. 8 SSB mRNA levels in HeLa cells treated with compound C8-1.
FIG. 9 SSB mRNA levels in HeLa cells treated with compound C10-7.
FIG. 10 SSB mRNA levels in HeLa cells treated with compound C10-8.
FIG. 11 SSB mRNA levels in rat retina.

To illustrate the invention via cartoon, shown herein is a modular composition, comprising an oligonucleotide ([O₁][O₂][O₃]......[Oₙ]), a linker(s) (L), a peptide(s) (P), and an optional lipid(s) (X), targeting ligand(s) (X), and/or solubilizing group(s) (X).
In an embodiment, the modular composition may have the formula:

P-L-[O₁][O₂][O₃]......[Oₙ]-L-P.

In another embodiment, the modular composition may have the formula:

P-L-[O₁][O₂][O₃]......[Oₙ]-X.

Examples of modular compositions are: 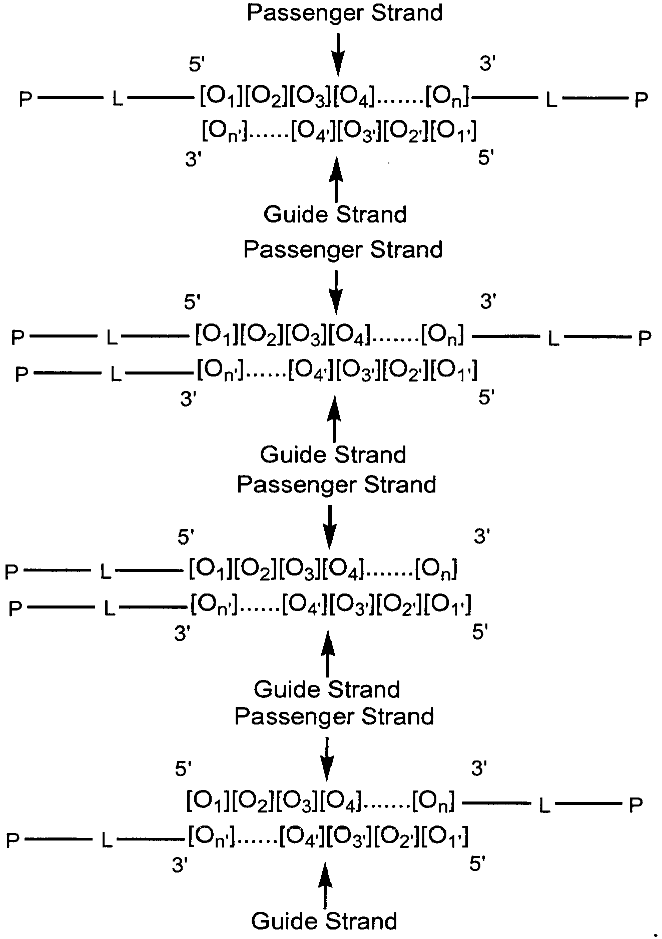 Another representation of a modular composition is:

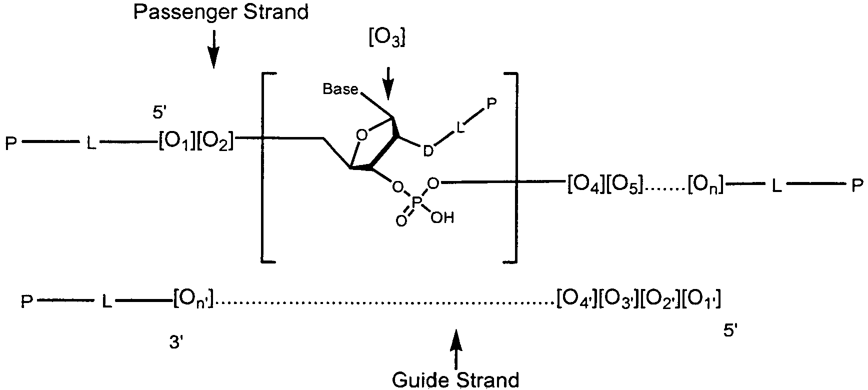

These examples are used as guidance. One skilled in the art will recognize that a variety of permutations for placing the desired components on the passenger and guide strand exist.

Any number of linkers, and therefore any number of peptides, can be attached to the oligonucleotide. A preferred range of numbers of linkers is from 1-8. A more preferred range of numbers of linkers is from 1-4. A preferred range of numbers of peptides is from 1-8. A more preferred range of numbers of peptides is from 1-4.

The two strands contain n and n' nucleotides respectively. The numbers n and n' can be equal or different. The numbers are integers ranging from 8 to 50. Preferably, the numbers are integers ranging from 12-28. More preferably, the numbers are integers ranging from 19-21.

As an example, each nucleotide [Oₙ] or [O_{n'}], that contains a linker (L-P and/or L-X) has generic structures shown in the following cartoon:

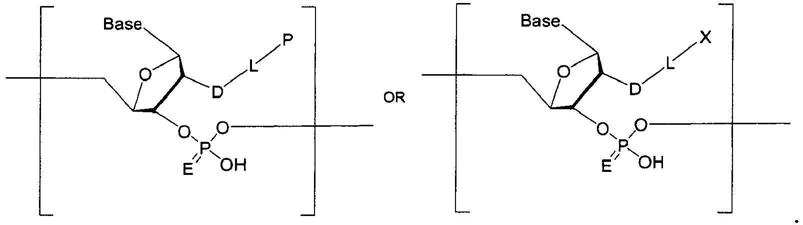

For each nucleotide, 1) E = oxygen (O) or sulfur (S); 2) Base = A, U, G or C, which can be modified or unmodified; 3) D is the connection point between ribose ring and linker L, D = oxygen (O), sulfur (S, S(O) or S(O)₂), nitrogen (N-R, wherein R = H, alkyl, L-P or L-X), carbon (CH-R, wherein R = H, alkyl, L-P, or L-X), or phosphorus (P(O)R or P(O)(OR), wherein R = alkyl, L-P, or L-X). Preferably, D = oxygen (O).

The two nucleotides [Oₙ₋₁] and [Oₙ] or [O_{n'-1}] and [O_{n'}] are connected via phosphodiester or thio-phosphodiester bonds.

When the oligonucleotide is a double-stranded oligonucleotide, the "P-L" and the lipid, targeting ligand, and/or solubilizing group may be located on the same strand or on different strands.

In some embodiments, the "P-L" and the lipid, targeting ligand, and/or solubilizing group are on the same strand.

In some embodiments, the "P-L" and the lipid, targeting ligand, and/or solubilizing group are on the passenger strand.

In some embodiments, the "P-L" and the lipid, targeting ligand, and/or solubilizing group are on the guide strand.

In some embodiments, the "P-L" and the lipid, targeting ligand, and/or solubilizing group are located on different strands.

In some embodiments, the "P-L" is on the passenger strand while the lipid, targeting ligand, and/or solubilizing group is on the guide strand.

In some embodiments, the "P-L" and the lipid, targeting ligand, and/or solubilizing group are on different strands but on the same terminal end of the double-stranded oligonucleotide.

In some embodiments, the "P-L" and the lipid, targeting ligand, and/or solubilizing group are on different strands and on the opposite terminal ends of the double-stranded oligonucleotide.

In some embodiments, an additional "P-L" of identical or different nature can be used in place of the lipid, targeting ligand, and/or solubilizing group noted in the above embodiments.

In some embodiments, the "P-L" can be located on multiple terminal ends of either the passenger or guide strand and the the lipid, targeting ligand, and/or solubilizing group can be located on the remaining terminal ends of the passenger and guide strands. In some embodiments, one "P-L" and two or more lipids, targeting ligands, and/or solubilizing groups are present in the oligonucleotide.

In some embodiments, two or more "P-L" and two or more lipids, targeting ligands and/or solubilizing groups are present in the oligonucleotide.

In some embodiments, when the oligonucleotide is a double-stranded oligonucleotide and multiple "P-L" components and/or lipids, targeting ligands, and/or solubilizing groups are present, such multiple "P-L" components and/or lipids, targeting ligands, and/or solubilizing groups may all be present in one strand or both strands of the double stranded oligonucleotide.

When multiple "P-L" components and/or lipids, targeting ligands, and/or solubilizing groups are present, they may all be the same or different.

In some embodiments, the "P-L" are on internal nucleotides only (i.e. excluding the 3'- and 5'-terminal ends of the oligonucleotide).

Described herein is a method of delivering an oligonucleotide to a cell. The method includes (a) providing or obtaining a modular composition of the invention; (b) contacting a cell with the modular composition; and (c) allowing the cell to internalize the modular composition.

The method can be performed in vitro, ex vivo or in vivo, e.g., to treat a subject identified as being in need of an oligonucleotide, e.g., a human, in need of having the expression of a gene or genes, e.g., a gene related to a disorder, downregulated or silenced.

In one aspect, described herein is a method for inhibiting the expression of one or more genes. The method comprises contacting one or more cells with an effective amount of an oligonucleotide, wherein the effective amount is an amount that suppresses the expression of the one or more genes. The method can be performed in vitro, ex vivo or in vivo.

The methods and compositions described herein, e.g., the modular composition described herein, can be used with any oligonucleotides known in the art. In addition, the methods and compositions described herein can be used for the treatment of any disease or disorder known in the art, and for the treatment of any subject, e.g., any animal, any mammal, such as a human. One of ordinary skill in the art will also recognize that the methods and compositions described herein may be used for the treatment of any disease that would benefit from downregulating or silencing a gene or genes.

The methods and compositions described herein, e.g., the modular composition described herein, may be used with any dosage and/or formulation described herein, or any dosage or formulation known in the art. In addition to the routes of administration described herein, an ordinarily skilled artisan will also appreciate that other routes of administration may be used to administer the modular composition of the invention.

### Oligonucleotide

An "oligonucleotide" as used herein, is a poly stranded, double stranded or single stranded, unmodified or modified RNA, PNA or DNA. Examples of modified RNAs include those which have greater resistance to nuclease degradation than do unmodified RNAs. Further examples include those which have a 2' sugar modification, a base modification, a modification in a single strand overhang, for example a 3' single strand overhang, or, particularly if single stranded, a 5' modification which includes one or more phosphate groups or one or more analogs of a phosphate group. Examples and a further discription of oligonucleotides can be found in WO2009/126933.

In an embodiment, an oligonucleotide is an antisense, miRNA or siRNA. The preferred oligonucleotide is an siRNA. Another preferred oligonuleotide is the passenger strand of an siRNA. Another preferred oligonucleotide is the guide strand of an siRNA. siRNA

siRNA directs the sequence-specific silencing of mRNA through a process known as RNA interference (RNAi). The process occurs in a wide variety of organisms, including mammals and other vertebrates. Methods for preparing and administering siRNA and their use for specifically inactivating gene function are known. siRNA includes modified and unmodified siRNA. Examples and a further discription of siRNA can be found in WO2009/126933.

A number of exemplary routes of delivery are known that can be used to administer siRNA to a subject. In addition, the siRNA can be formulated according to any exemplary method known in the art. Examples and a further discription of siRNA formulation and administration can be found in WO2009/126933.

### Peptides

For macromolecular drugs and hydrophilic drug molecules, which cannot easily cross bilayer membranes, entrapment in endosomal/lysosomal compartments of the cell is thought to be the biggest hurdle for effective delivery to their site of action. Without wishing to be bound by theory, it is believed that the use of peptides will facilitate oligonucleotide escape from these endosomal/lysosomal compartments or oligonucleotide translocation across a cellular membrane and release into the cytosolic compartment. In certain embodiments, the peptides may be polycationic or amphiphilic or polyanionic peptides or peptidomimetics which show pH-dependent membrane activity and/or fusogenicity. A peptidomimetic may be a small protein-like chain designed to mimic a peptide.

In some embodiments, the peptide is a cell-permeation agent, preferably a helical cell-permeation agent. These peptides are commonly referred to as Cell Penetrating Peptides. See, for example, "Handbook of Cell Penetrating Peptides" Ed. Langel, U.; 2007, CRC Press, Boca Raton, Florida. Preferably, the component is amphipathic. The helical agent is preferably an alpha-helical agent, which preferably has a lipophilic and a lipophobic phase. A cell-permeation agent can be, for example, a cell permeation peptide, cationic peptide, amphipathic peptide or hydrophobic peptide, e.g. consisting primarily of Tyr, Trp and Phe, dendrimer peptide, constrained peptide or crosslinked peptide. Examples of cell penetrating peptides include Tat, Penetratin, and MPG. For the present invention, it is believed that the cell penetrating peptides can be a "delivery" peptide, which can carry large polar molecules including peptides, oligonucleotides, and proteins across cell membranes. Cell permeation peptides can be linear or cyclic, and include D-amino acids, "retro-inverso" sequences, non-peptide or pseudo-peptide linkages, peptidyl mimics. In addition the peptide and peptide mimics can be modified, e.g. glycosylated, pegylated, or methylated. Examples and a further description of peptides can be found in WO2009/126933.

The peptides may be conjugated at either end or both ends by addition of a cysteine or other thiol containing moiety to the C- or N -terminus. When not functionalized on the N-terminus, peptides may be capped by an acetyl group, or may be capped with a lipid, a PEG, or a targeting moiety. When the C-terminus of the peptides is unconjugated or unfunctionalized, it may be capped as an amide, or may be capped with a lipid, a PEG, or a targeting moiety.

The peptides described herein are:
HFHHFFHHFFHFFHHFFHHF (SEQ ID NO: 1);
WHHWWHWWHHWWHHW (SEQ ID NO: 2);
HWHHLLHHLLHLLHHLLHHL (SEQ ID NO: 3);
HLHHWLHHLLHLLHHLLHHL (SEQ ID NO: 4);
HLHHLWHHLLHLLHHLLHHL (SEQ ID NO: 5);
HLHHLLHHLWHLLHHLLHHL (SEQ ID NO: 6);
HLHHLLHHLLHWLHHLLHHL (SEQ ID NO: 7);
HLHHLLHHLLHLLHHWLHHL (SEQ ID NO: 8);
HLHHLLHHLLHLLHHLWHHL (SEQ ID NO: 9);
HPHHLLHHLLHLLHHLLHHL (SEQ ID NO: 10);
HLHHPLHHLLHLLHHLLHHL (SEQ ID NO: 11);
HLHHLPHHLLHLLHHLLHHL (SEQ ID NO: 12);
HLHHLLHHLPHLLHHLLHHL (SEQ ID NO: 13);
HLHHLLHHLLHLLHHLPHHL (SEQ ID NO: 14);
HLHHLLHHLLHLLHHLLHHP (SEQ ID NO: 15);
ELEELLEELLHLLHHLLHHL (SEQ ID NO: 16);
ELHHLLHELLHLLHELLHHL (SEQ ID NO: 17);
GLWRALWRLLRSLWRLLWRAC (SEQ ID NO: 18);
GLFEAIEGFIENGWEGMIDGWYGYGRKKRRQRR (SEQ ID NO: 19);
HLHHLLHHLLHLLHHLLHHL (SEQ ID NO: 20);
HWHHWWHHWWHWWHHWWHHW (SEQ ID NO: 21);
HLHHLLHHWLHLLHHLLHHL (SEQ ID NO: 22);
HLHHLLHHLLHLWHHLLHHL (SEQ ID NO: 23);
HLHHLLHHLLHLLHHLLHHW (SEQ ID NO: 24);
HHHHHHHHHHLLLLLLLLLL (SEQ ID NO: 25);
HHHHHHHLLLLLLL (SEQ ID NO: 26);
LTTLLTLLTTLLTTL (SEQ ID NO: 27);
KLLKLLKLWLKLLKLLLKLL (SEQ ID NO: 28);
LHLLHHLLHHLHHLLHHLLHLLHHLLHHL (SEQ ID NO: 29);
FLGGIISFFKRLF (SEQ ID NO: 30);
FIGGIISFIKKLF (SEQ ID NO: 31);
FIGGIISLIKKLF (SEQ ID NO: 32);
HLLHLLLHLWLHLLHLLLHLL (SEQ ID NO: 33);
GIGGAVLKVLTTGLPALISWIKRKRQQ (SEQ ID NO: 34);
RQIKIWFQNRRMKWKKGG (SEQ ID NO: 35);
RKKRRQRRRPPQ (SEQ ID NO: 36);
GALFLGWLGAAGSTMGAPKKKRKV (SEQ ID NO: 37);
GGGARKKAAKAARKKAAKAARKKAAKAARKKAAKAAK (SEQ ID NO: 38);
GWTLNSAGYLLGKINLKALAALAKKIL (SEQ ID NO: 39);
RRRRRRRRR (SEQ ID NO: 40);
WEAKLAKALAKALAKHILAKALAKALKACEA (SEQ ID NO: 41);
WEAALAEALAEALAEHLAEALAEAEALEALAA (SEQ ID NO: 42);
D(NHC12H25)NleKNleKNleHNleKNleHNle (SEQ ID NO: 43);
KLLKLLLKLWLKLLKLLLKLL (SEQ ID NO: 44);
GLFEAIAGFIENGWEGMIDGWYG (SEQ ID NO: 45);
GLFHAIAAHFIHGGWHGLIHGWYG (SEQ ID NO: 46);
GLFEAIAEFIEGGWEGLIEGWYG (SEQ ID NO: 47);
GLFEAIEGFIENGWEGMIDGWYG (SEQ ID NO: 48);
GLFKAIAKFIKGGWKGLIKGWYG (SEQ ID NO: 49);
GLFEAIAGFIENGWEGMIDGWYGYGRKKRRQRR (SEQ ID NO: 50);
GLFEAIAGFIENGWEGMIDGWYGRQIKIWFQNRRMKWKKGG (SEQ ID NO: 51);
GLFHAIAAHFIHGGWHGLIHGWYGYGRKKRRQRR (SEQ ID NO: 52);
GLFEAIAEFIEGGWEGLIEGWYGYGRKKRRQRR (SEQ ID NO: 53);
GLFEAIEGFIENGWEGMIDGWYGYGRKKRRQRR (SEQ ID NO: 54);
GLFKAIAKFIKGGWKGLIKGWYGYGRKKRRQRR (SEQ ID NO: 55);
GFFALIPKIISSPLFKTLLSAVGSALSSSGEQE (SEQ ID NO: 56);
LHLLHHLLHHLHHLLHHLLHLLHHLLHHLGGGRKKRRQRRRPPQ (SEQ ID NO: 57);
RKKRRQRRRPPQGGGLHLLHHLLHHLHHLLHHLLHLLHHLLHHL (SEQ ID NO: 58); and
LIRLWSHIHIWFQWRRLKWKKK (SEQ ID NO:59);
wherein the peptides are optionally conjugated at either end by addition of a cysteine or other thiol containing moiety to the C- or N-terminus; or when not functionalized on the N-terminus, the peptides are optionally capped by an acetyl group, lipid, peg or a targeting moiety; or when not functionalized on the C-terminus, the peptides are optionally capped by an amide, lipid, peg or a targeting moiety.

The preferred peptides (P) are:
GLFEAIEGFIENGWEGMIDGWYGYGRKKRRQRR (SEQ ID NO: 19);
KLLKLLLKLWLKLLKLLLKLL (SEQ ID NO: 28);
LHLLHHLLHHLHHLLHHLLHLLHHLLHHL (SEQ ID NO: 29);
HLLHLLLHLWLHLLHLLLHLL (SEQ ID NO: 33);
RKKRRQRRRPPQ (SEQ ID NO: 36);
RQIKIWFQNRRMKWKKGG (SEQ ID NO: 40);
GLFEAIAGFIENGWEGMIDGWYGYGRKKRRQRR (SEQ ID NO: 50);
GLFEAIAGFIENGWEGMIDGWYGRQIKIWFQNRRMKWKKGG (SEQ ID NO: 51);
GLFHAIAAHFIHGGWHGLIHGWYGYGRKKRRQRR (SEQ ID NO: 52);
GLFEAIAEFIEGGWEGLIEGWYGYGRKKRRQRR (SEQ ID NO: 53);
GLFEAIEGFIENGWEGMIDGWYGYGRKKRRQRR (SEQ ID NO: 54);
GLFKAIAKFIKGGWKGLIKGWYGYGRKKRRQRR (SEQ ID NO: 55);
GFFALIPKIISSPLFKTLLSAVGSALSSSGEQE (SEQ ID NO: 56);
LHLLHHLLHHLHHLLHHLLHLLHHLLHHLGGGRKKRRQRRRPPQ (SEQ ID NO: 57);
RKKRRQRRRPPQGGGLHLLHHLLHHLHHLLHHLLHLLHHLLHHL (SEQ ID NO: 58); and
LIRLWSHIHIWFQWRRLKWKKK (SEQ ID NO:59);
wherein the peptides are optionally conjugated at either end by addition of a cysteine or other thiol containing moiety to the C- or N-terminus; or when not functionalized on the N-terminus, the peptides are optionally capped by an acetyl group, lipid, peg or a targeting moiety; or when not functionalized on the C-terminus, the peptides are optionally capped by an amide, lipid, peg or a targeting moiety.

### Linkers

The covalent linkages between the peptide and the oligonucleotide of the modular composition described herein is mediated by a linker. This linker may be cleavable or non-cleavable, depending on the application. In certain embodiments, a cleavable linker may be used to release the oligonucleotide after transport from the endosome to the cytoplasm. The intended nature of the conjugation or coupling interaction, or the desired biological effect, will determine the choice of linker group. Linker groups may be combined or branched to provide more complex architectures. Examples and a further discription of linkers can be found in WO2009/126933.

The linkers described herein are shown in Table 1:

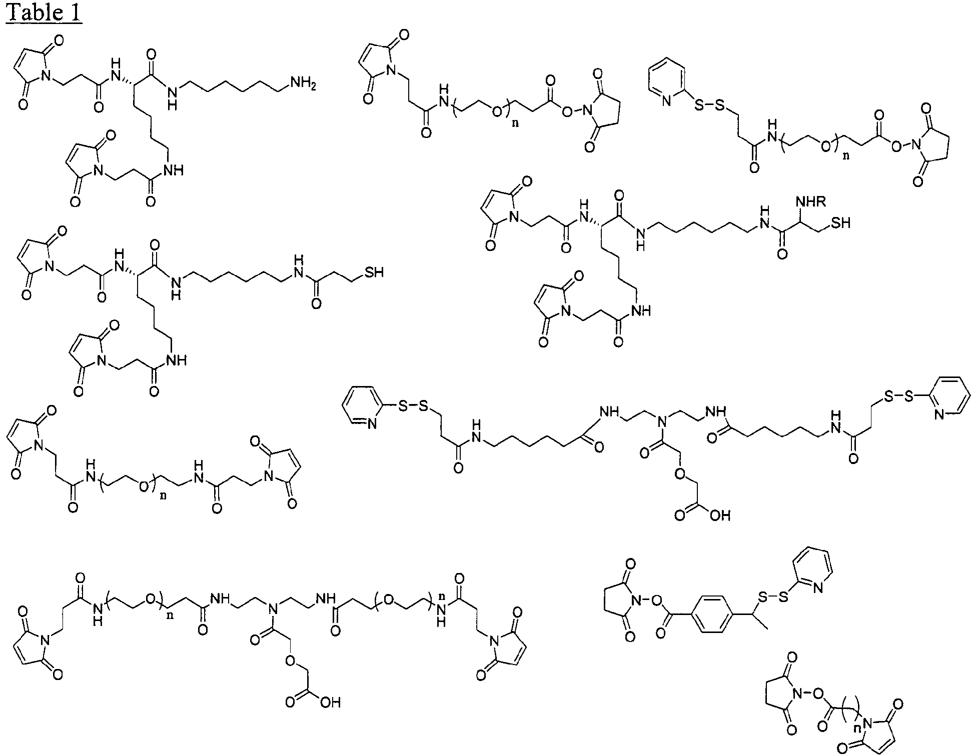

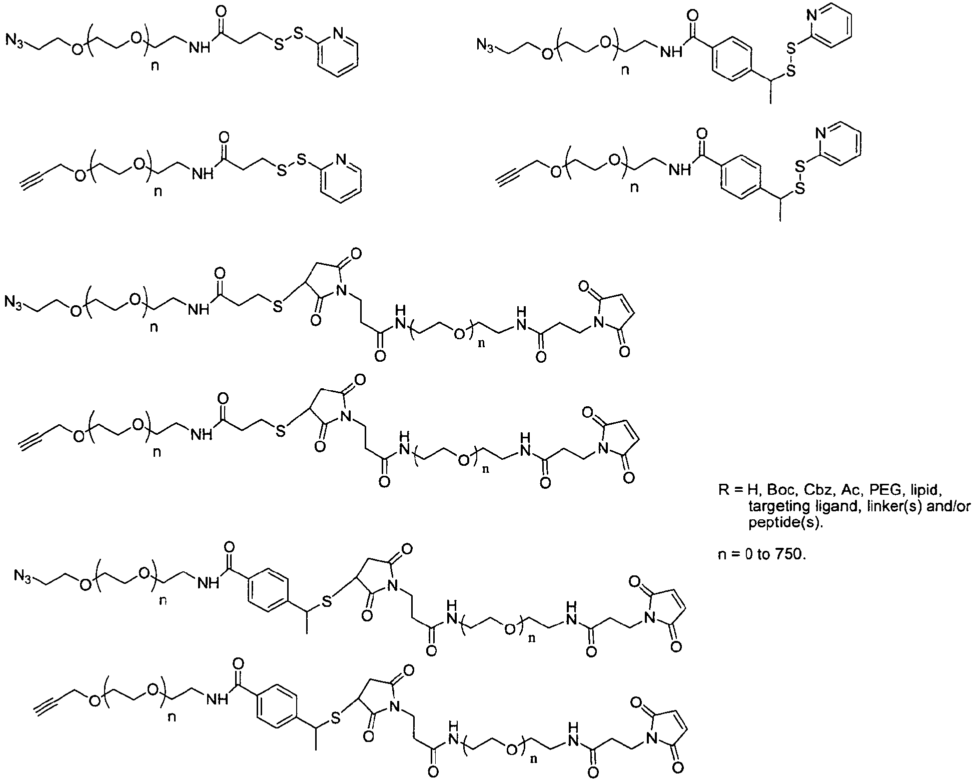

The preferred linkers are shown in Table 2:

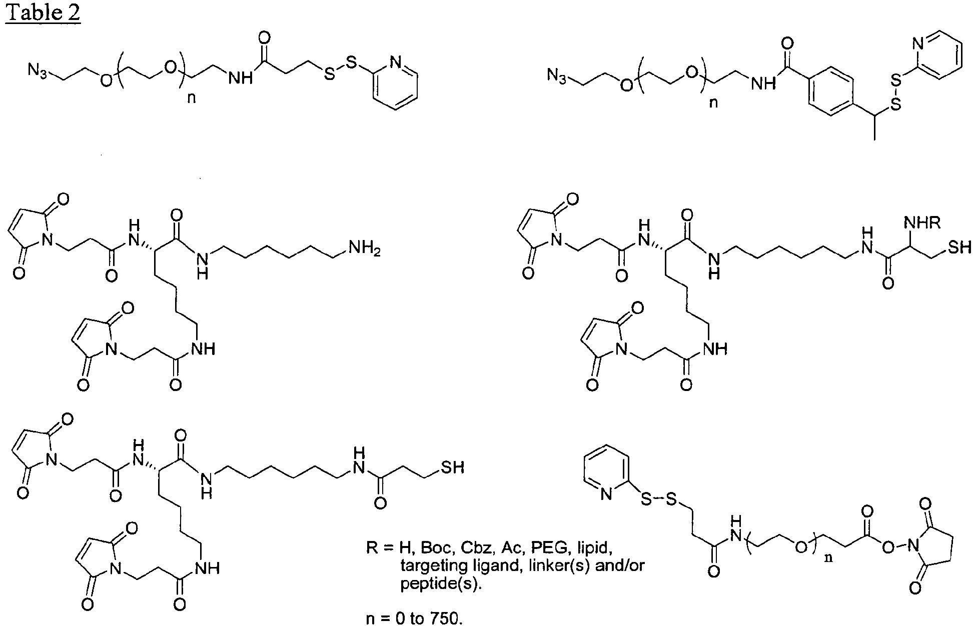

Commercial linkers are available from various suppliers such as Pierce or Quanta Biodesign including combinations of said linkers. The linkers may also be combined to produce more complex branched architectures accomodating from 1 to 8 peptides as illustrated in one such example below:

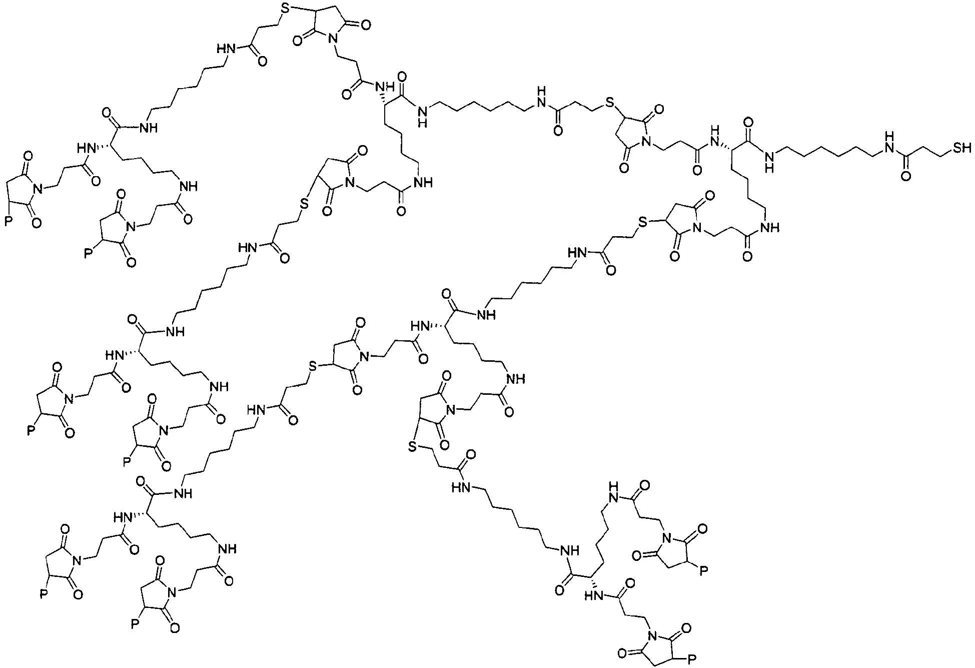

### Targeting Ligands

The modular compositions described herein may comprise a targeting ligand. In some embodiments, this targeting ligand may direct the modular composition to a particular cell. For example, the targeting ligand may specifically or non-specifically bind with a molecule on the surface of a target cell. The targeting moiety can be a molecule with a specific affinity for a target cell. Targeting moieties can include antibodies directed against a protein found on the surface of a target cell, or the ligand or a receptor-binding portion of a ligand for a molecule found on the surface of a target cell. Examples and a further discription of targeting ligands can be found in WO2009/126933.

The targeting ligands are selected from the group consisting of an antibody, a ligand-binding portion of a receptor, a ligand for a receptor, an aptamer, D-galactose, N-acetyl-D-galactose (GalNAc), multivalent N-acytyl-D-galactose, D-mannose, cholesterol, a fatty acid, a lipoprotein, folate, thyrotropin, melanotropin, surfactant protein A, mucin, carbohydrate, multivalent lactose, multivalent galactose, N-acetyl-galactosamine, N-acetyl-glucosamine, multivalent mannose, multivalent fructose, glycosylated polyaminoacids, transferin, bisphosphonate, polyglutamate, polyaspartate, a lipophilic moiety that enhances plasma protein binding, a steroid, bile acid, vitamin B12, biotin, an RGD peptide, an RGD peptide mimic, ibuprofen, naproxen, aspirin, folate, and analogs and derivatives thereof.

The preferred targeting ligands are selected from the group consisting of an RGD peptide, an RGD peptide mimic, D-galactose, N-acetyl-D-galactosamine (GalNAc),

### GalNAc₂, and GalNAc₃, cholesterol, folate, and analogs and derivatives thereof. Lipids

Lipophilic moieties, such as cholesterol or fatty acids, when attached to highly hydrophilic molecules such as nucleic acids can substantially enhance plasma protein binding and consequently circulation half life. In addition, lipophilic groups can increase cellular uptake. For example, lipids can bind to certain plasma proteins, such as lipoproteins, which have consequently been shown to increase uptake in specific tissues expressing the corresponding lipoprotein receptors (e.g., LDL-receptor or the scavenger receptor SR-B1). Lipophilic conjugates can also be considered as a targeted delivery approach and their intracellular trafficking could potentially be further improved by the combination with endosomolytic agents.

Exemplary lipophilic moieties that enhance plasma protein binding include, but are not limited to, sterols, cholesterol, fatty acids, cholic acid, lithocholi.c acid, dialkylglycerides, diacylglyceride, phospholipids, sphingolipids, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, 03-(oleoyl)cholenic acid, dimethoxytrityl, phenoxazine, aspirin, naproxen, ibuprofen, vitamin E and biotin etc. Examples and a further discription of lipids can be found in WO2009/126933.

Examples of lipids include:

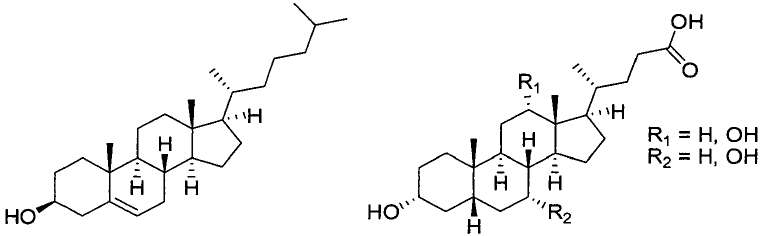

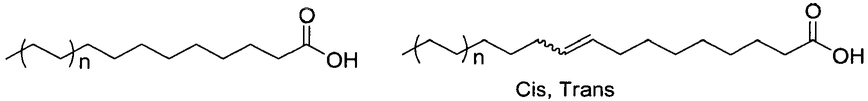

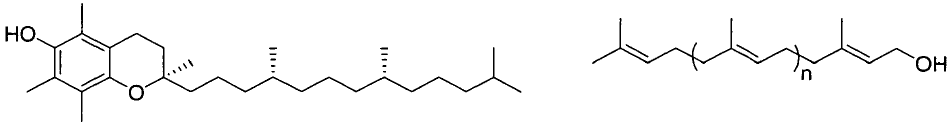

The preferred lipid is cholesterol.

### Solubilizing Agents

The modular composition may comprise one or more other moieties/ligands that may enhance aqueous solubility, circulation half life and/or cellular uptake. These can include naturally occurring substances, such as a protein (e.g., human serum albumin (HSA), low-density lipoprotein (LDL), high-density lipoprotein (HDL), or globulin); or a carbohydrate (e.g,, a dextran, pullulan, chitin, chitosan, inulin, cyclodextrin or hyaluronic acid). These moieties may also be a recombinant or synthetic molecule, such as a synthetic polymer or synthetic polyamino acids. Examples include polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG, e.g., PEG-0.5K, PEG-2K, PEG-5K, PEG-10K, PEG-12K, PEG-15K, PEG-20K, PEG-40K), methyl-PEG (mPEG), [mPEG]2, polyvinyl alcohol (PVA), polyurethane, poly(2 ethylacryllic acid), N-isopropylacrylamide polymers, or polyphosphazine. Examples and a further discription of solubilizing agents can be found in WO2009/126933.

The preferred solubilizing group is PEG 0.5K to 30K.

### Method of Treatment

Described herein is a method of treating a subject at risk for or afflicted with a disease that may benefit from the administration of the modular composition of the invention. The method comprises administering the modular composition of the invention to a subject in need thereof, thereby treating the subject. The oligonucleotide that is administered will depend on the disease being treated. For example, conjugates of the instant invention are useful for the treatment of cancer. See WO2009/126933 for additional details regarding methods of treatments for specific indications.

### Formulation

There are numerous methods for preparing conjugates of oligonucleotide compounds. The techniques should be familiar to those skilled in the art. A useful reference for such reactions is Bioconjugate Techniques, Hermanson, G. T., Academic Press, San Diego, CA, 1996. Other references include WO2005/041859; WO2008/036825 and WO2009/126933.

### EXAMPLES

The invention is further illustrated by the following examples, which should not be construed as further limiting. The siRNAs described herein were designed to target the ubiquitously expressesd gene SSB (Sjogren syndrome antigen B; NM_009278.4).

Oligonucleotide synthesis is well known in the art. (See US patent applications: US 2006/0083780, US 2006/0240554, US 2008/0020058, US 2009/0263407 and US 2009/0285881 and PCT patent applications: WO 2009/086558, WO2009/127060, WO2009/132131, WO2010/042877, WO2010/054384, WO2010/054401, WO2010/054405 and WO2010/054406). The siRNAs disclosed and utilized in the Examples were synthesized via standard solid phase procedures.

Linker groups may be connected to the oligonucleotide strand(s) at a linkage attachment point (LAP) and may include any carbon-containing moiety, in some embodiments having at least one oxygen atom, at least one phosphorous atom, and/or at least one nitrogen atom. In some embodiments, the phosphorous atom forms part of a terminal phosphate, or phosphorothioate group on the linker group, which may serve as a connection point for the oligonucleotide strand. In certain embodiments, the nitrogen atom forms part of a terminal ether, ester, amino or amido (NHC(O)-) group on the linker group, which may serve as a connection point for the linkers of interest, endosomolytic unit, cell penetrating peptide, solubilizing group, lipid, targeting group, or additional linkers of interest. These terminal linker groups include, but are not limited to, a C₆ hexyl, C₅ secondary-hydroxy, C₃ thiol or C₆ thiol moiety. An example from the RNA sequences described below is C₆ hexyl: [(CH₂)₆ NH₂].

### EXAMPLE 1

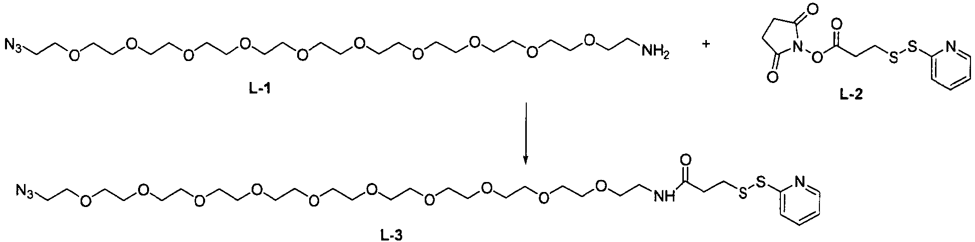

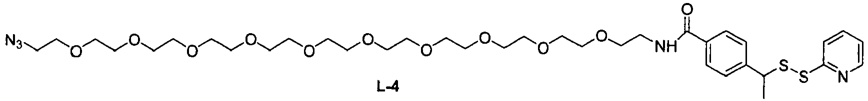

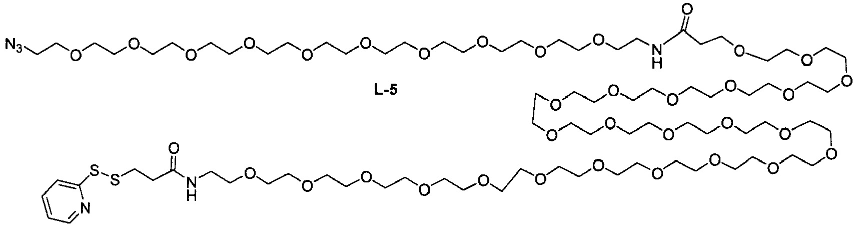

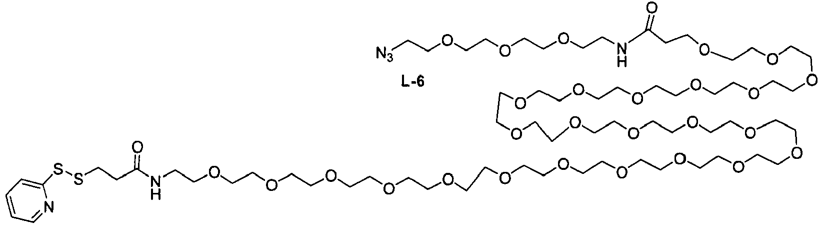

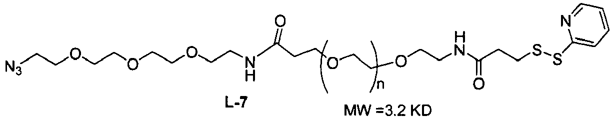

To a solution of NHS ester L-2 (100.0 mg, 0.320 mmol) in 0.5 mL anhydrous DCE was added azido amine L-1 (253.0 mg, 0.480 mmol) in 0.5 mL anhydrous DCE, followed by addition of 1.5 eq. trietthylamine. The resulting solution was stirred for 1 h at room temperature, and the reaction mixture was loaded on a silica column, eluding with MeOH/DCM=0/100 to 10/90 over 25 min. The collected fraction of L-3 was subject to LC-MS analysis and the result indicated the product was >95% pure.

Following the analogous procedures, azido disulfide L-4 to L-6 were prepared in >95% HPLC purity, L-7 was prepared from polydispersed SPDP-PEG-NHS ester.

### EXAMPLE 2

Crude, oligonucleotide R-1 15 mg was treated with azido-peg9-SPDP L-3 (25.3 mg, 0.035 mmol) and CuBr·Me₂S (0.760 mg, 3.70 µmοl) in 3 mL of DMA/Water=3/1. The resulting reaction mixture was stirred for 48 h at room temperature followed by addition of 2.0 mL of 40% NH₄F/water=1/1. The biphase mixture was stirred at 65 °C for 1 h, then purified by C₁₈ cartridges to give a crude white solid R-2 ∼ 5 mg.

Following the analogous procedures, RNA disulfides R-3 - R-11 were prepared respectively.

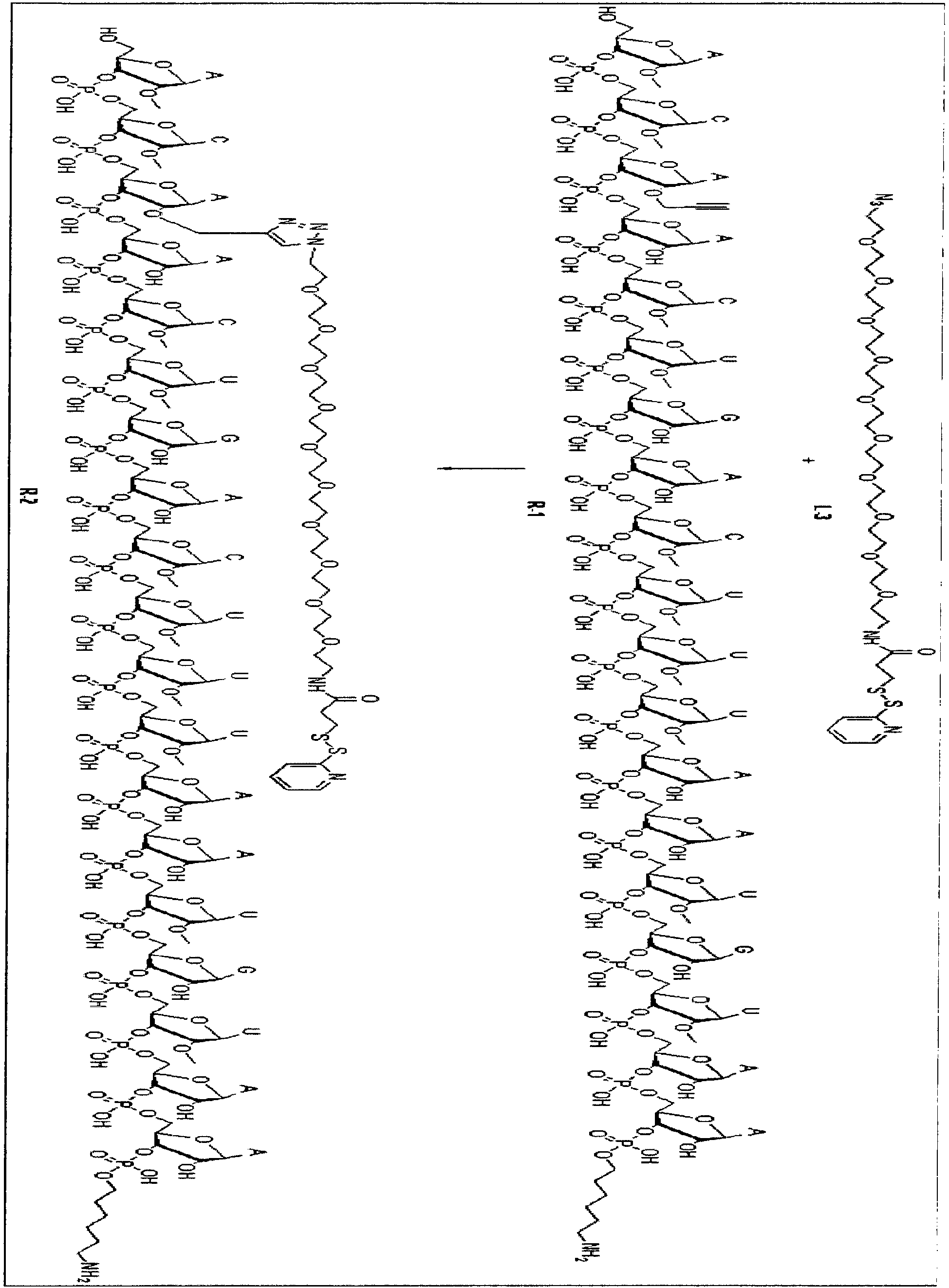

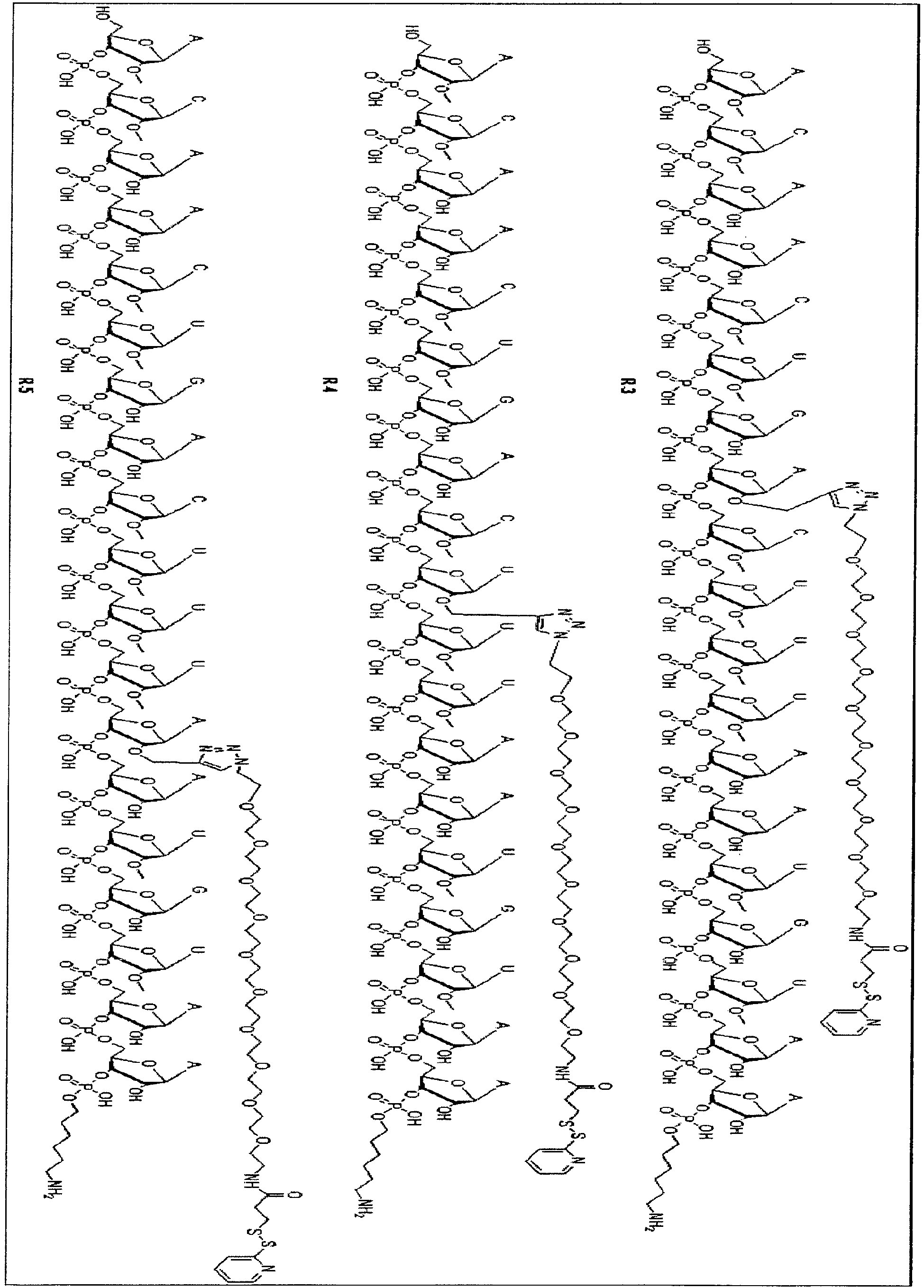

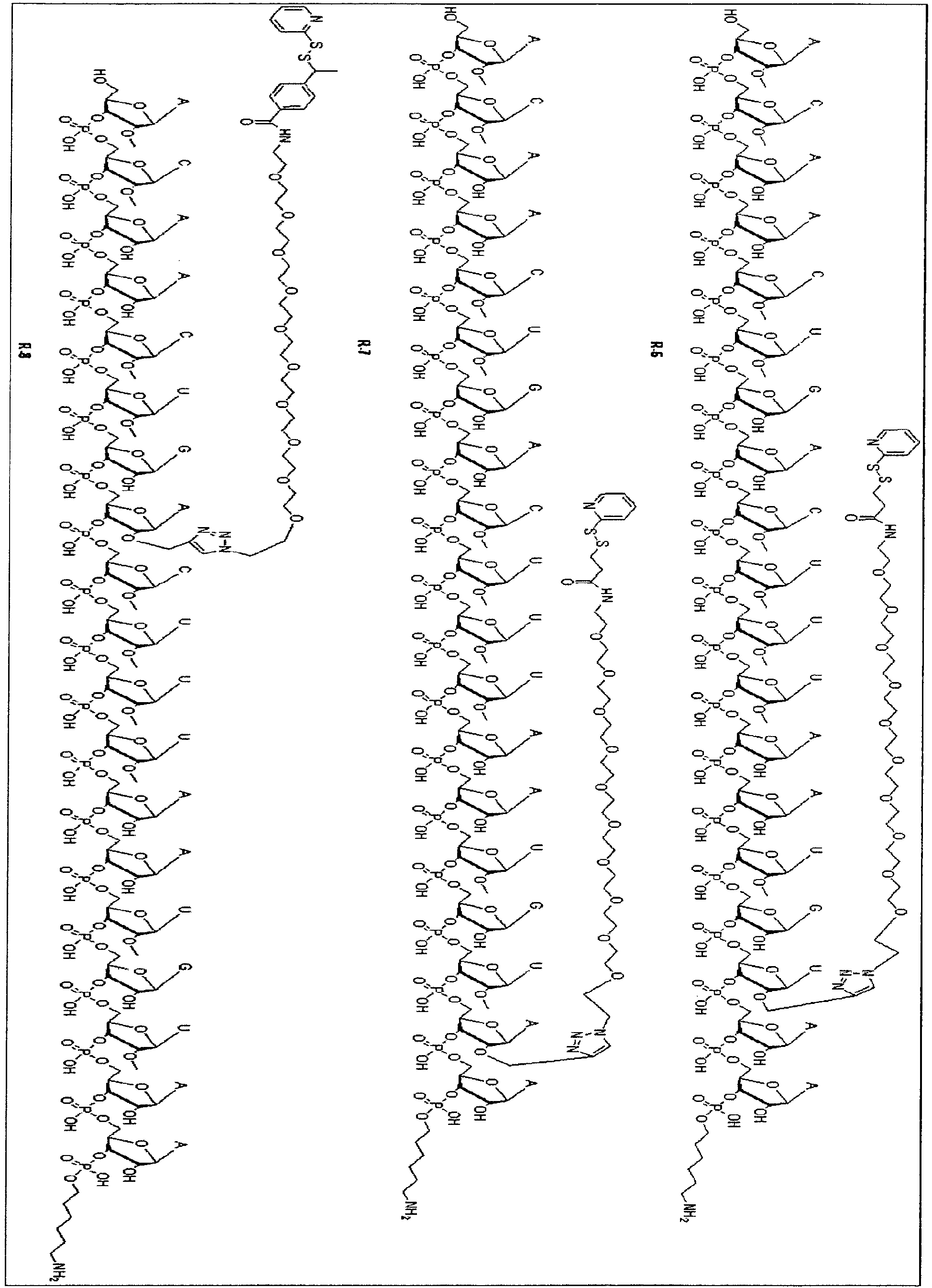

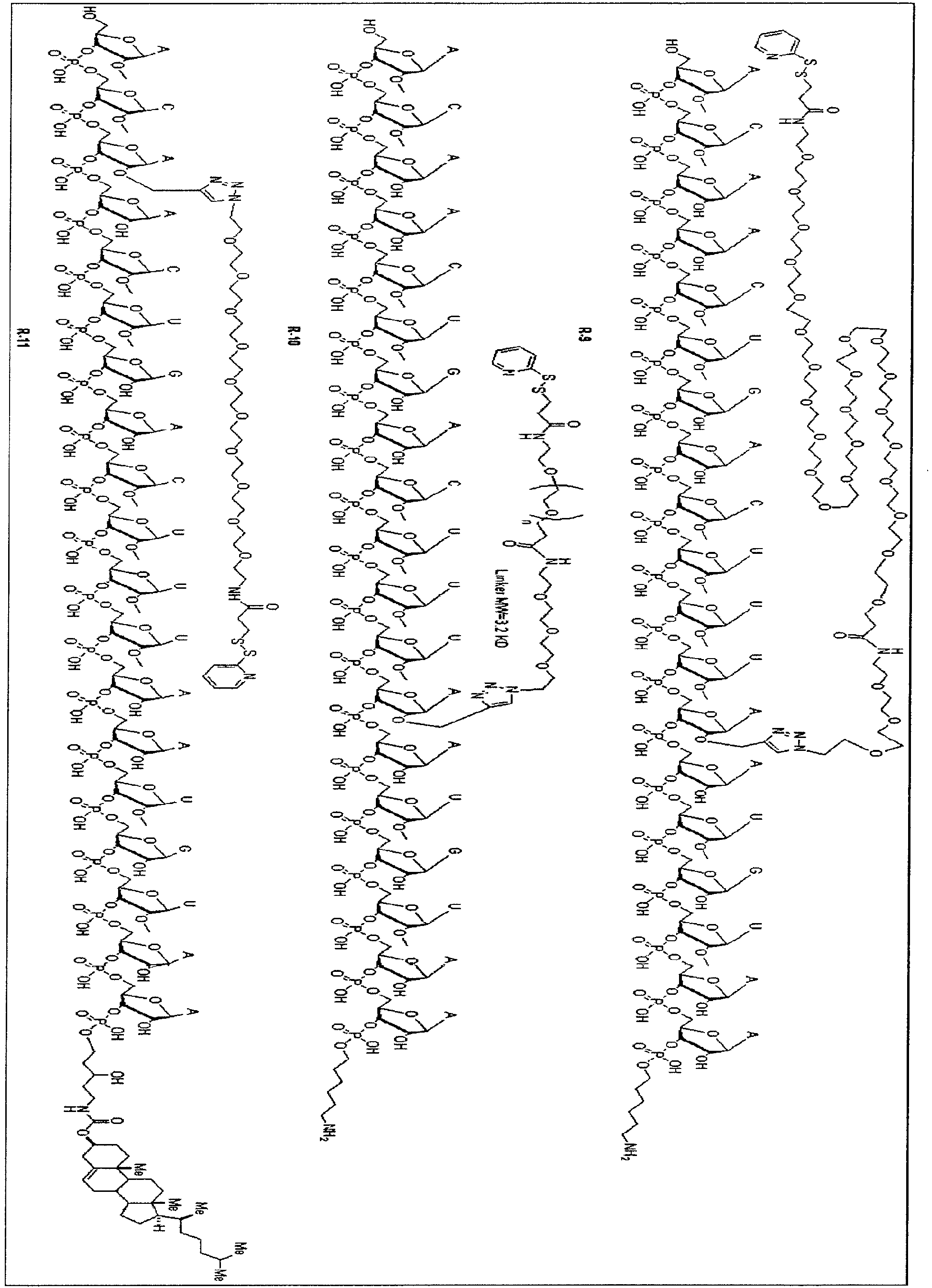

### EXAMPLE 3

Crude, oligonucleotide R-12 50 mg was treated with azido-peg9-SPDP L-3 (40.0 mg, 0.055 mmol) and CuBr·Me₂S (2.50 mg, 12 µmol) in 4 mL of DMA/Water=3/1. The resulting reaction mixture was stirred for 48 h at room temperature followed by addition of 2.0 mL of 40% NH₄F/water=1/1. The biphase mixture was stirred at 65°C for 1 h, then purified by C18 cartridges to give a crude white solid R-13 ∼ 15 mg.

Following the analogous procedures, RNA disulfides R-14 - R-18 were prepared respectively.

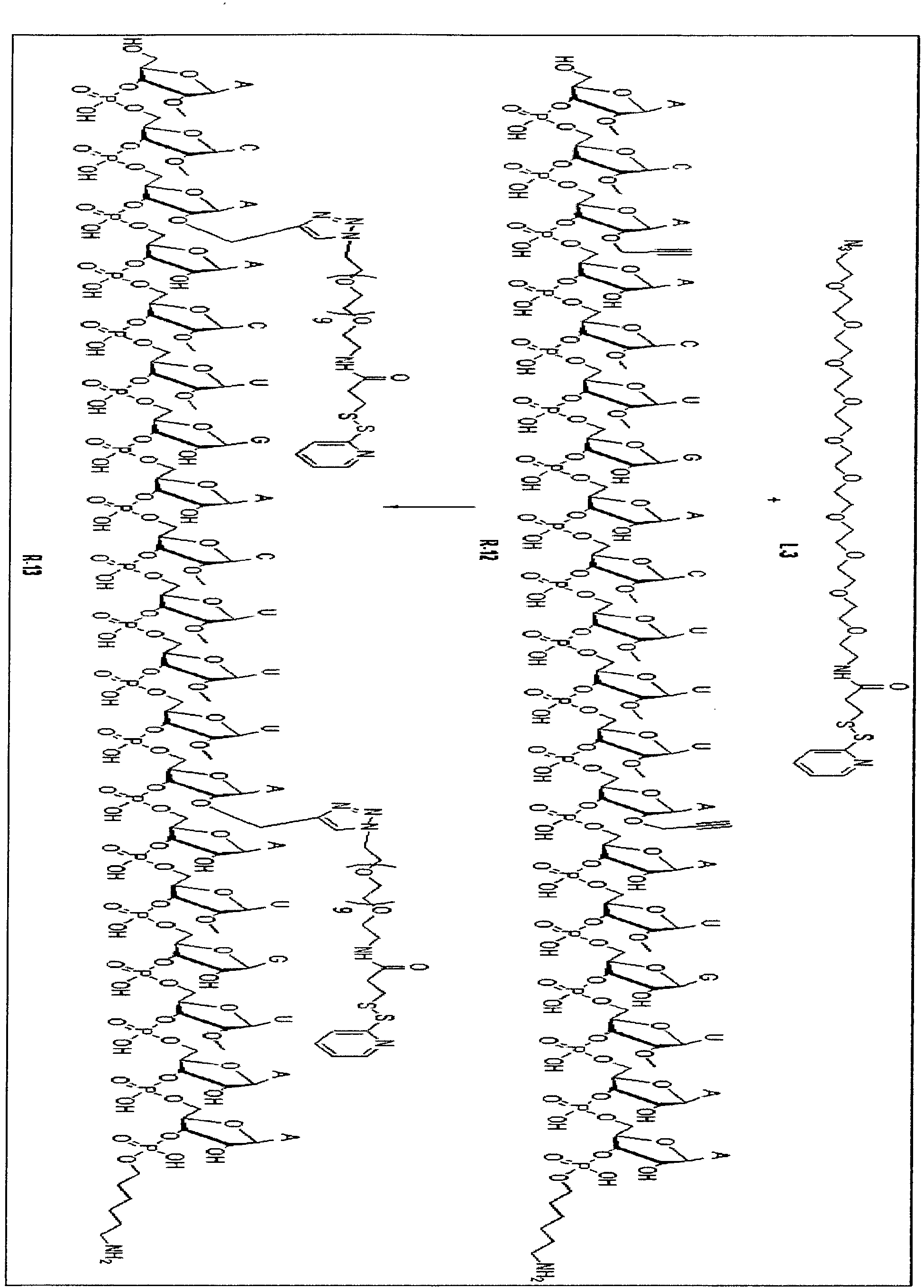

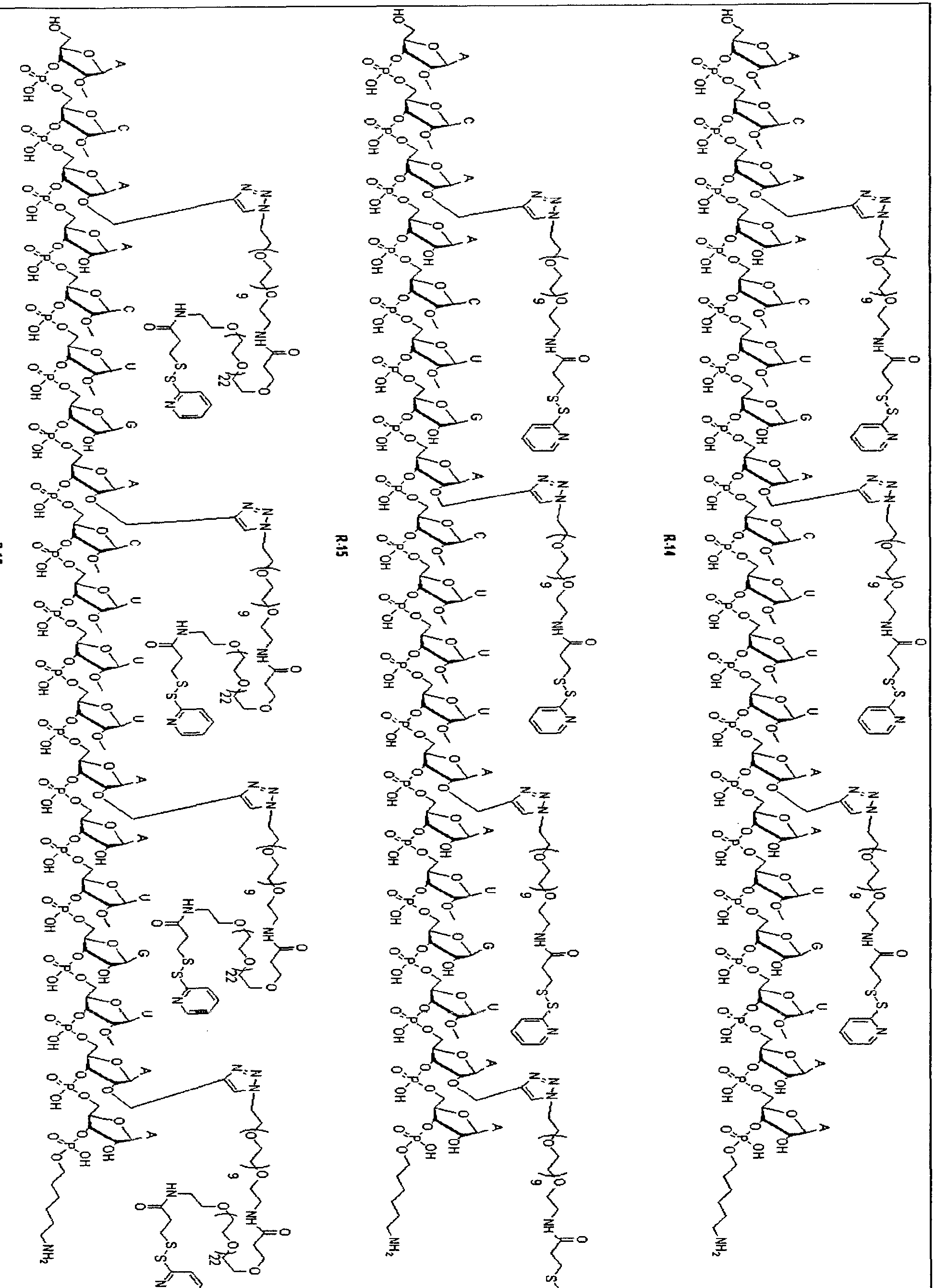

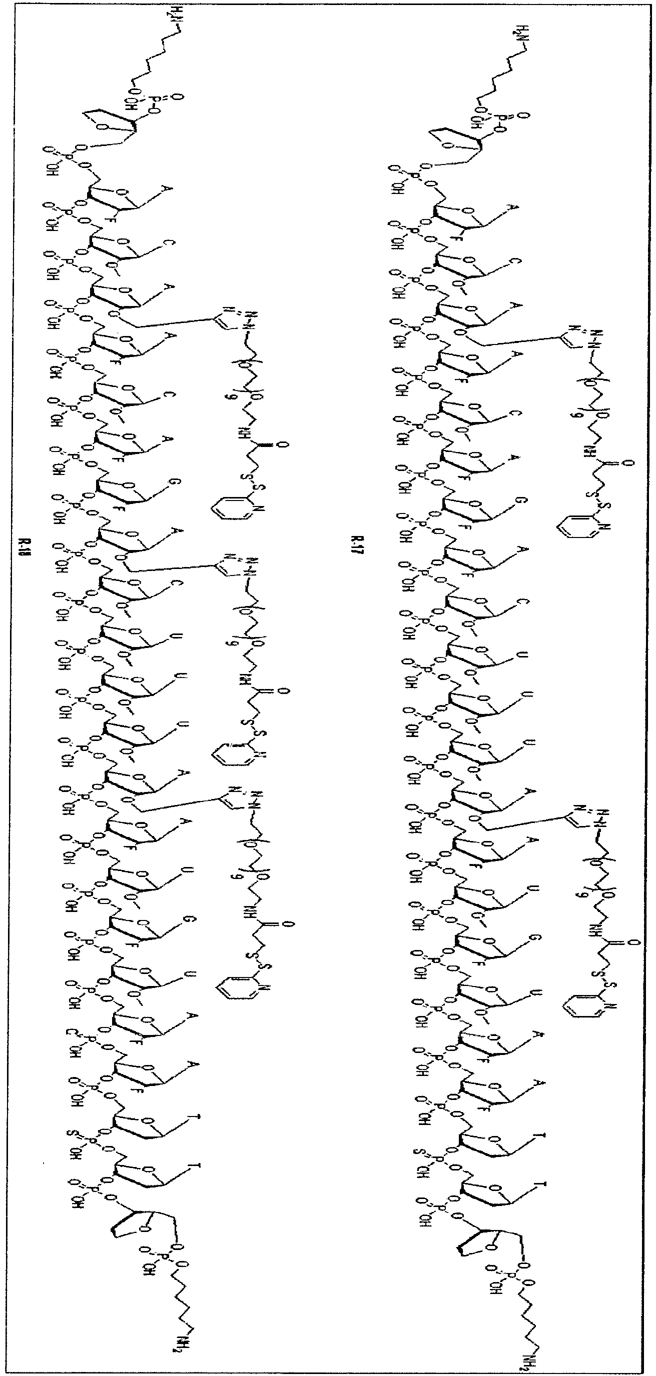

### EXAMPLE 4

Compound R-2 (1.50 mg, 0.211 µmol) in 400 µL formamide/pH=6.8 Tris buffer=3/1 was treated with peptide SEQ ID NO: 19 (1.724 mg, 0.423 µmol) in 400 µL of the same buffer and the resulting reaction mixture was stirred for 1 h. The reaction was diluted by addition of formamide/pH=6.8 Tris buffer=3/1 to a total volume of 2.5 mL and purified by strong anion exchange chromatography on a Resource Q column (25-75% B in A, A: formamide/H₂O=1/1, 20 mmol Tris·HCl, pH=7.4, B: formamide/H₂O=1/1, 20 mmol Tris·HCl, 400 mmol NaClO₄, pH=7.4). Combined product fractions were diluted with water, and centrifugally dialyzed 4 times against water with MW 10,000 cutoff membrane. The dialyte was lyophilized to provide R-19 as a white solid, mass = 11063.

Equal molar amount of guide strand G1 was mixed with compound R-19 to produce the corresponding double strand duplex C4-1. The duplex integrity was checked by CE analysis and the conjugate was submitted for biological evaluations.

Following the analogous procedures, RNA disulfide conjugates C4-2 to C4-14 were prepared respectively and submitted for biological evaluations.

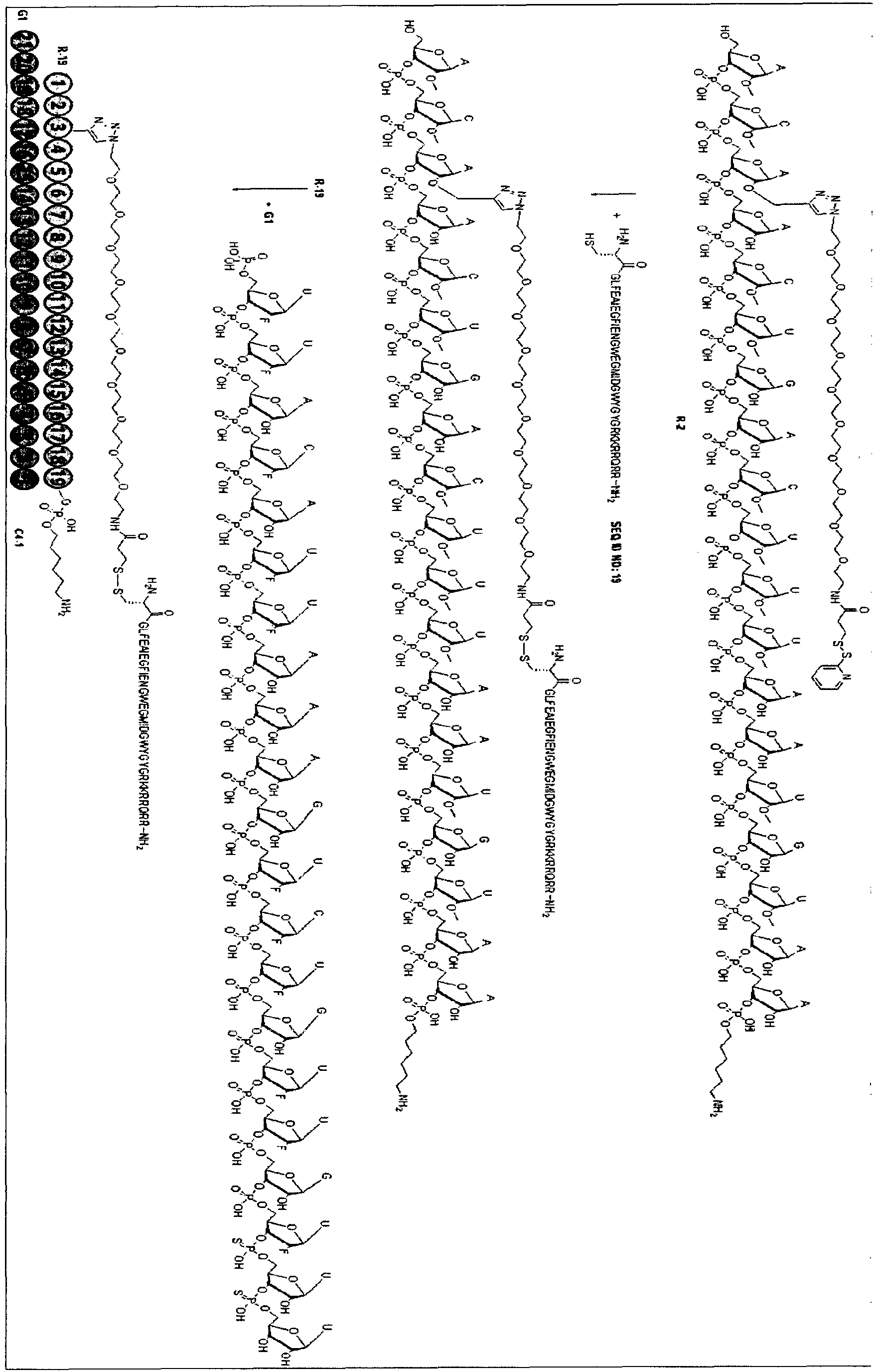

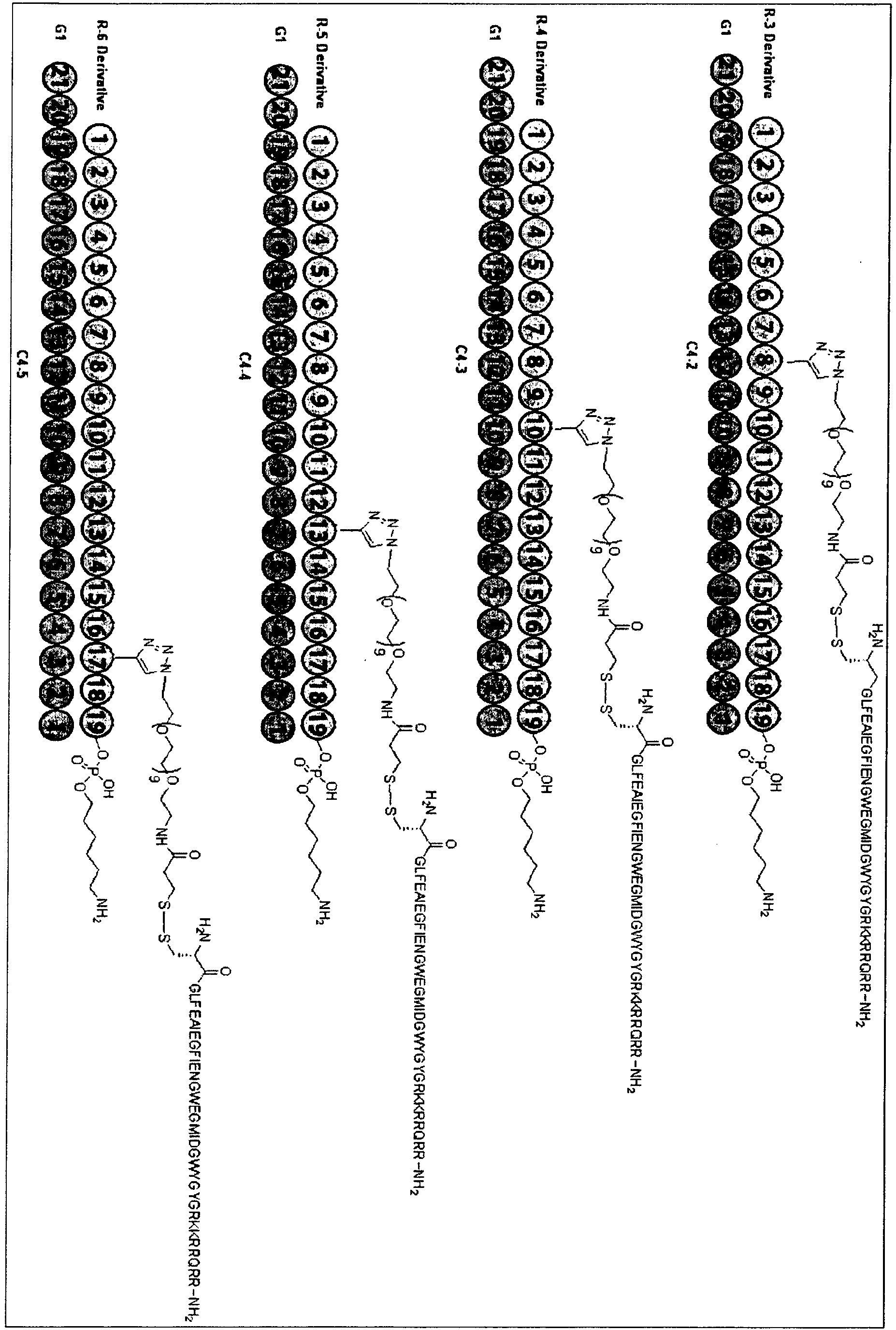

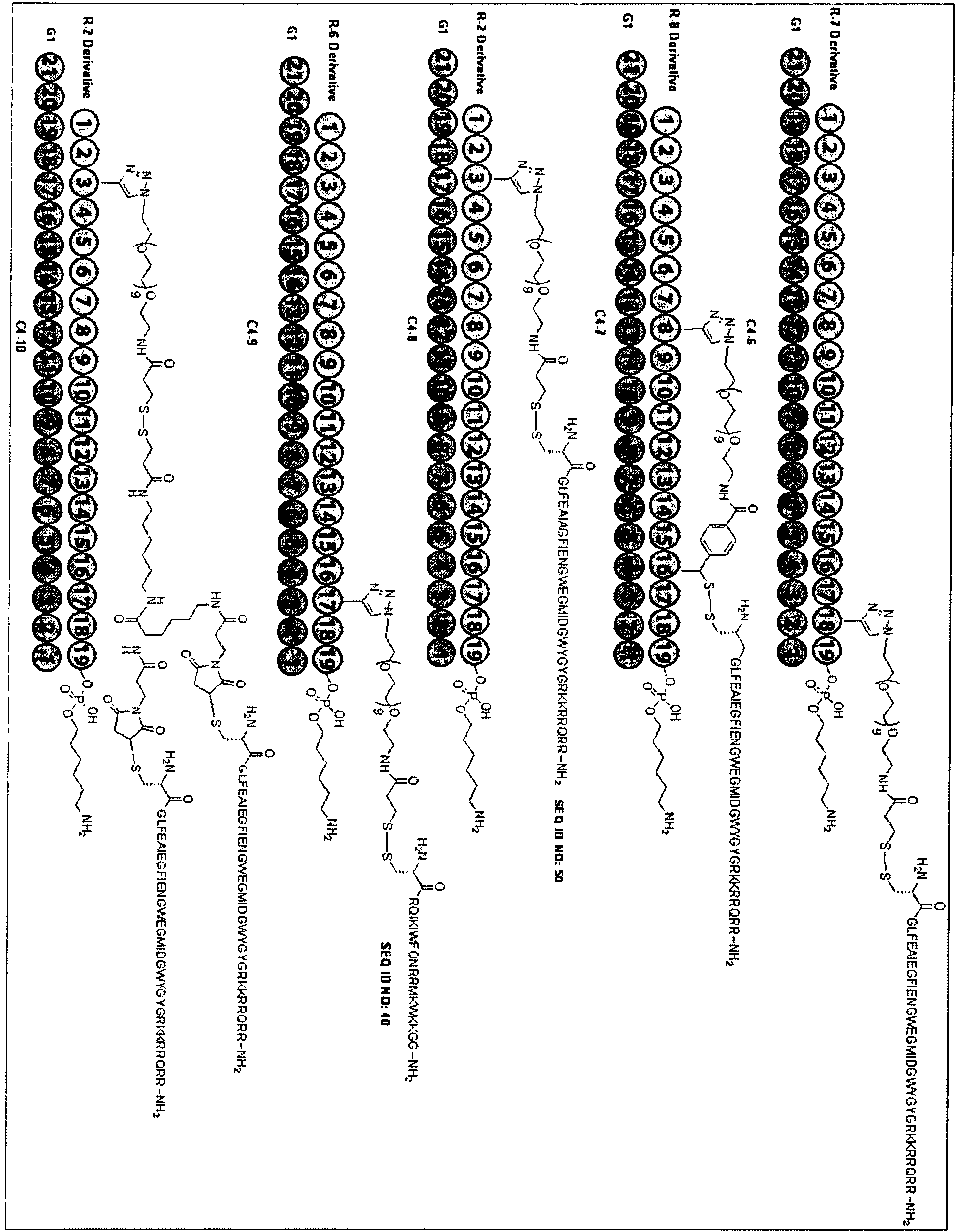

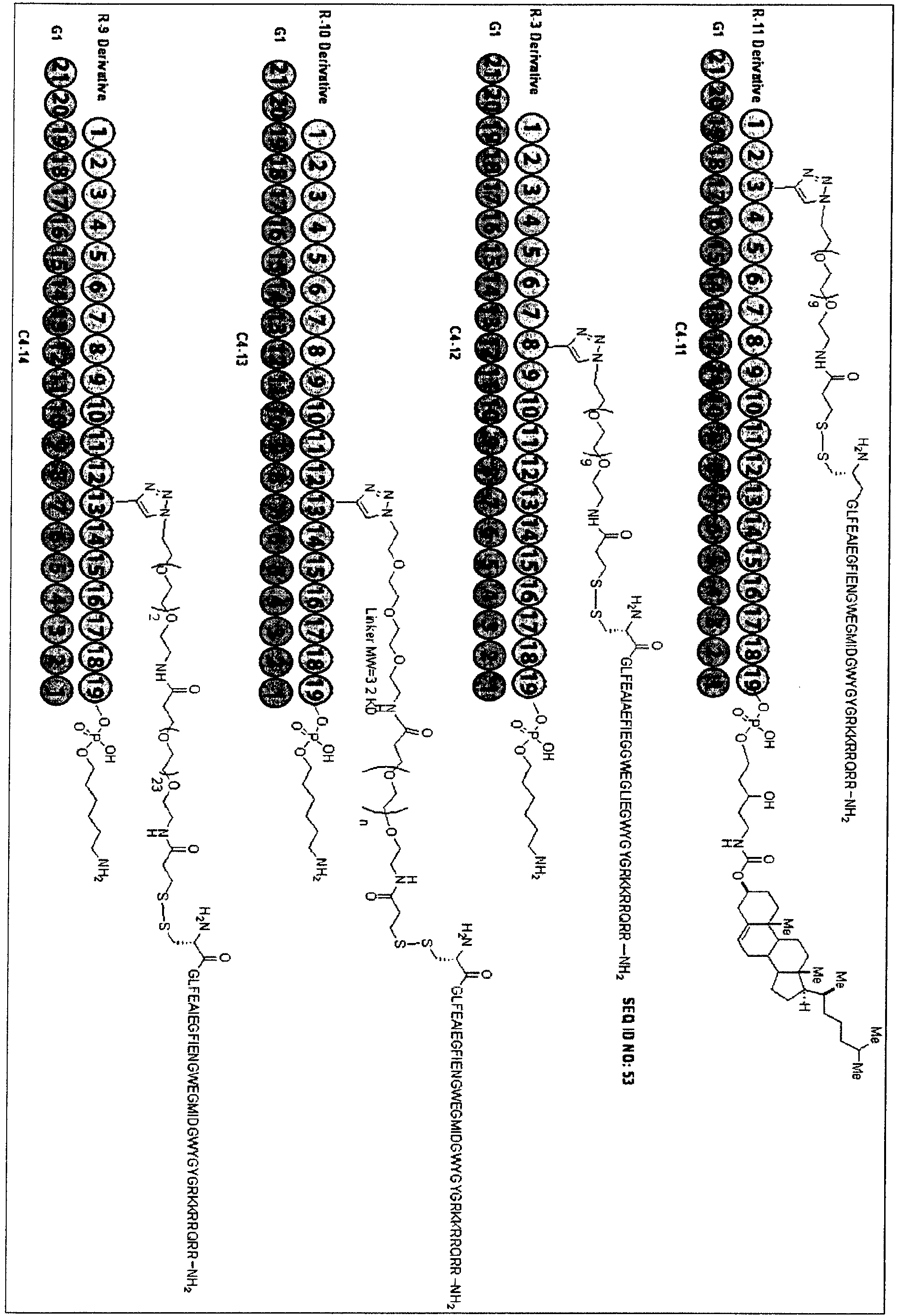

### EXAMPLE 5

Peptide SEQ ID NO: 19 (10.0 mg, 2.45 µmοl) was disolved in 500 µL pH=6.5 TEAA buffer and added dropwisely to linker L-8 (38.4 mg, 0.074 mmol) in 500 µL TEAA pH=6.5 buffer. The reaction was stirred for 2 h and purified by RP HPLC 10-90% MeCN/water over 20 min. The collected fractions were lyophilized to give a white solid SEQ ID NO: 19-1 which was > 95% pure by LC-MS analysis.

Reactant R-3 (2.00 mg, 0.282 µmol) and TCEP (0.808 mg, 2.82 µmοl) were dissolved in pH=6.8 Tris buffer 0.5 mL and stirred for 2 h. LC-MS trace indicated the cleavage of R-3 disulfide bond, then the reaction mixture was loaded onto a PD-10 desalting column. The collected fractions were lyophilized to give white solid R-20 and used for the next reaction without further purification.

Compound R-20 (2.00 mg, 0.286 µmol) in 300 µL formamide/pH=6.8 Tris buffer=3/1 was treated with peptide SEQ ID NO: 19-1 (3.95 mg, 0.859 µmol) in 300 µL of the same buffer and the resulting reaction mixture was stirred for 1 h. The reaction was diluted by addition of formamide/pH=6.8 Tris buffer=3/1 to a total volume of 2.5 mL and purified by strong anion exchange chromatography on a Resource Q column (25-75% B in A, A: formamide/H₂O=1/1, 20 mmol Tris·HCl, pH=7.4, B: formamide/H₂O=1/1, 20 mmol Tris·HCl, 400 mmol NaClO₄, pH=7.4). Combined product fractions were diluted with water, and centrifugally dialyzed 4 times against water with MW 10,000 cutoff membrane. The dialyte was lyophilized to provide R-21 (0.71 mg, 21.4%, >95% purity) as a white solid.

Equal molar amount of guide strand G1 was mixed with compound R-21 to produce the corresponding double strand duplex C5-1. The duplex integrity was checked by CE analysis and the conjugate was submitted for biological evaluations.

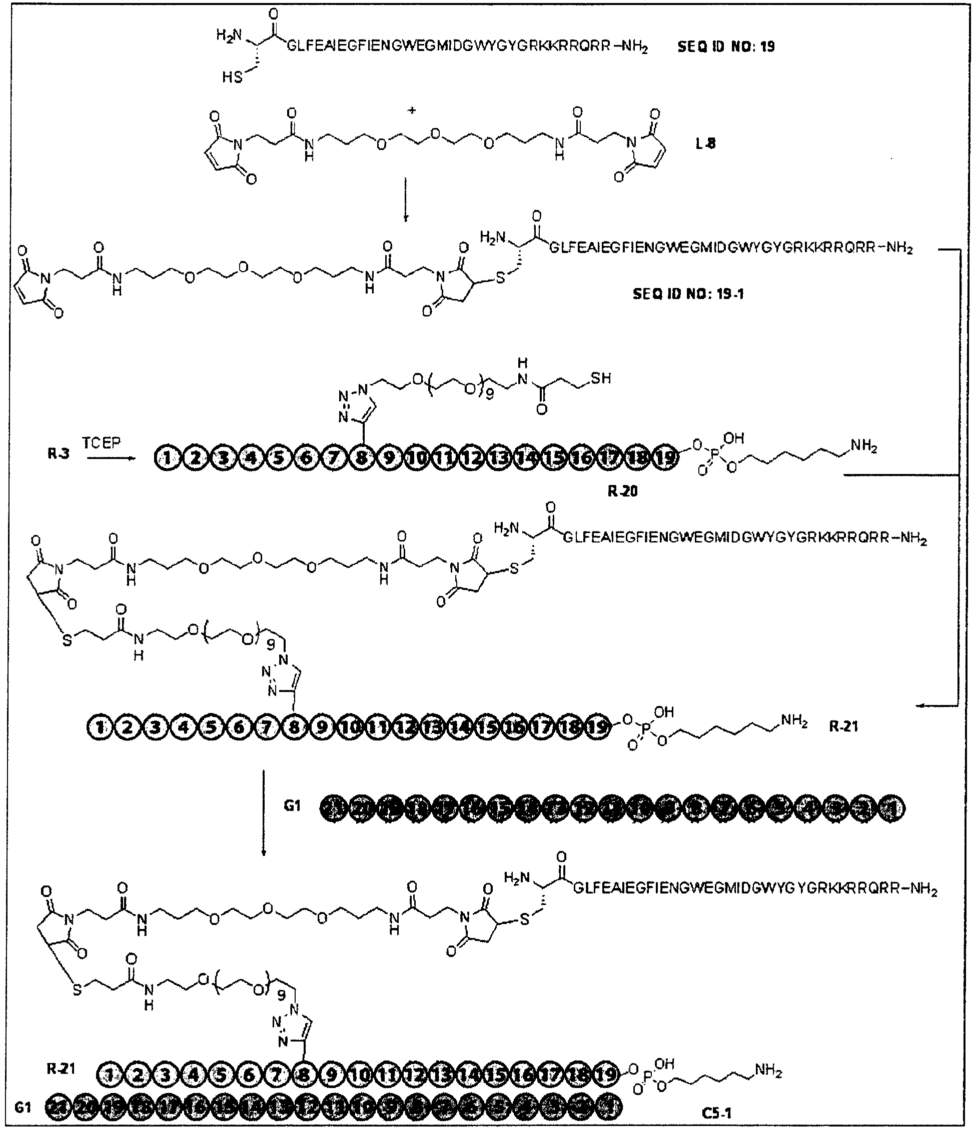

### EXAMPLE 6

Compound R-13 (3.00 mg, 0.382 µmol) in 300 µL formamide/pH=6.8 Tris buffer=3/1 was treated with peptide SEQ ID NO: 19 (4.98 mg, 1.222 µmol) in 300 µL of the same buffer and the resulting reaction mixture was stirred for 1 h. The reaction was diluted by addition of formamide/pH=6.8 Tris buffer=3/1 to a total volume of 2.5 mL and purified by strong anion exchange chromatography on a Resource Q column (25-75% B in A, A: formamide/H₂O=1/1, 20 mmol Tris·HCl, pH=7.4, B: formamide/H₂O=1/1, 20 mmol Tris·HCl, 400 mmol NaClO₄, pH=7.4). Combined product fractions were diluted with water, and centrifugally dialyzed 4 times against water with MW 10,000 cutoff membrane. The dialyte was lyophilized to provide R-22 (>90% purity) as a white solid.

Equal molar amount of guide strand G1 was mixed with compound R-22 to produce the corresponding double strand duplex C6-1. The duplex integrity was checked by CE analysis and the conjugate was submitted for biological evaluations.

Following the analogous procedures, RNA disulfide conjugates C6-2 to C6-6 were prepared and submitted for biological evaluations.

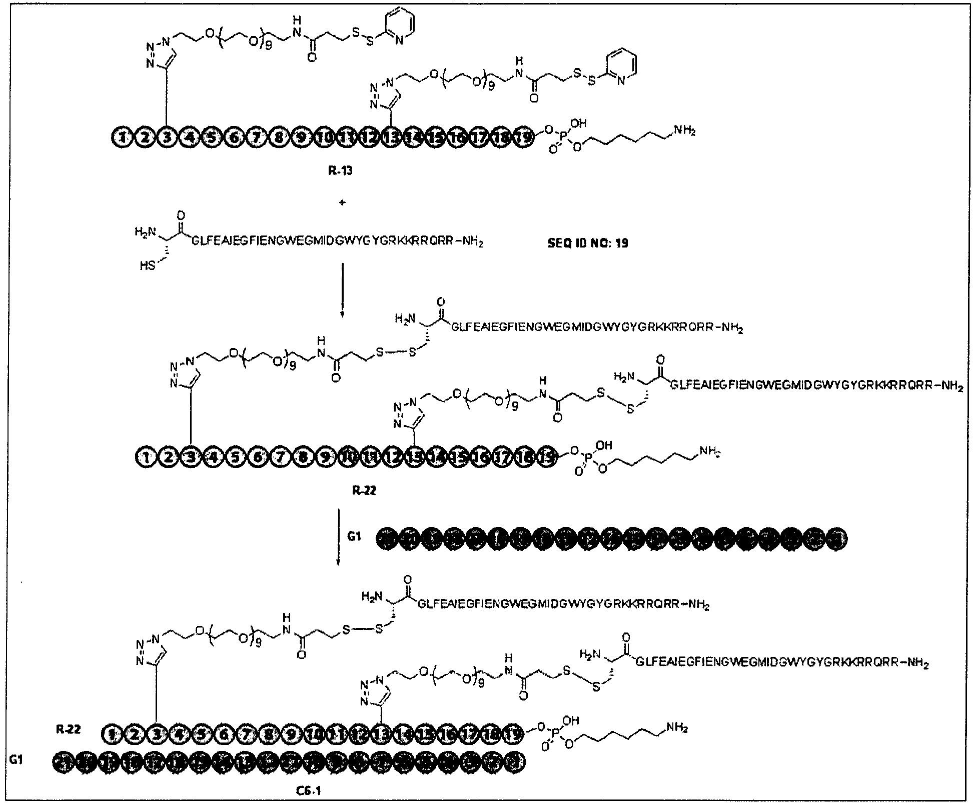

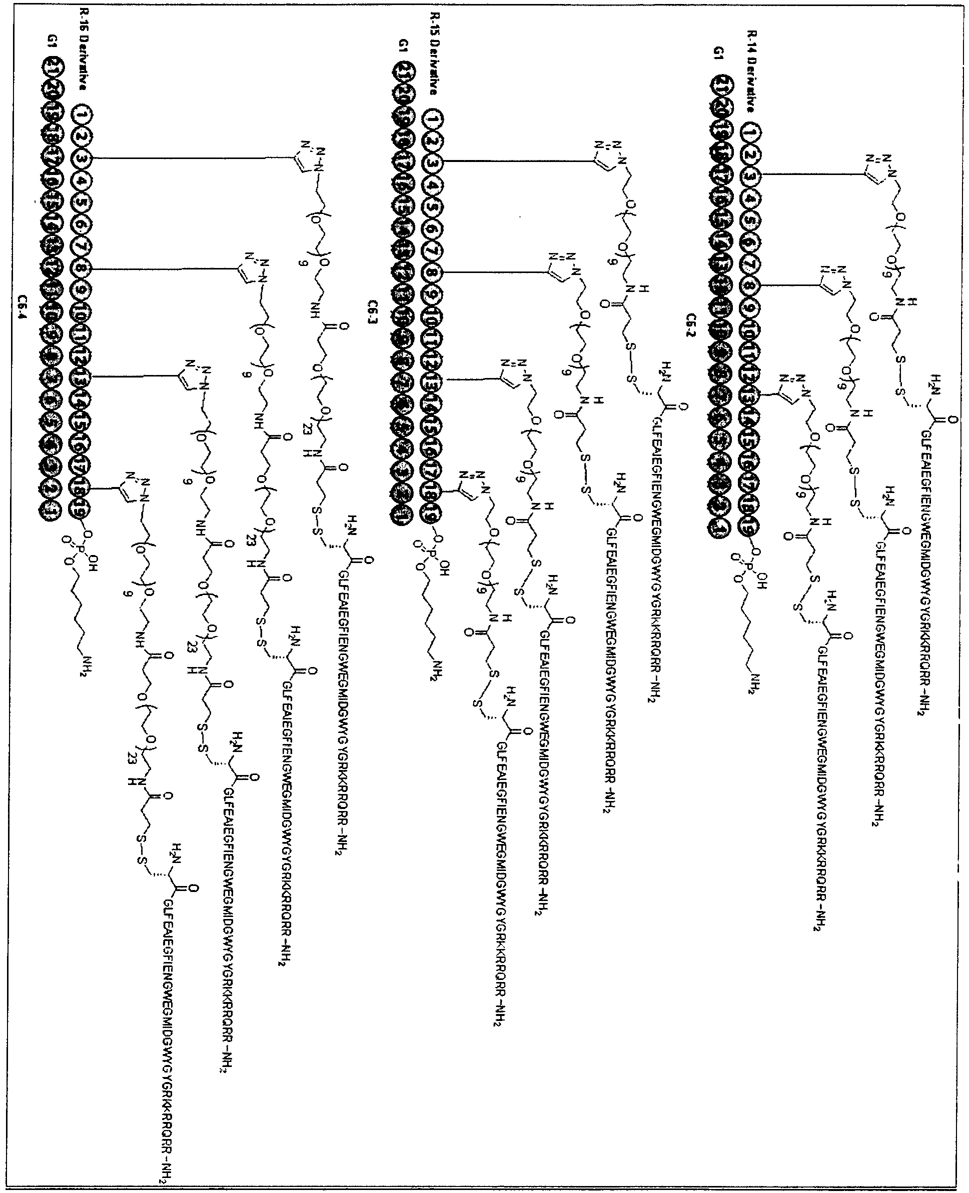

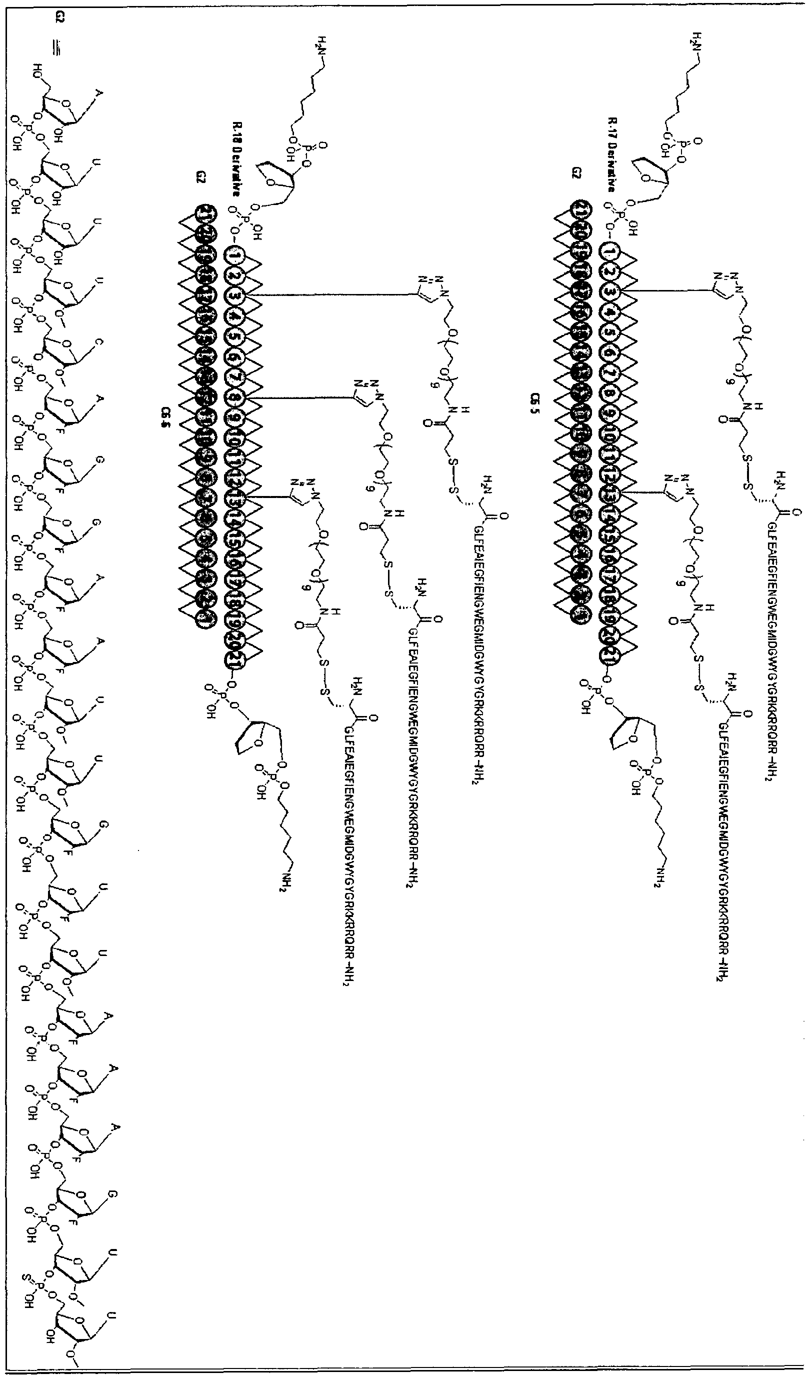

### EXAMPLE 7

Cystamine (1.13 g, 5.02 mmol) and cholesterol chloroformate (4.96 g, 11.04 mmol) were dissolved in DCM 20 mL, followed by addition of TEA (3.50 ml, 25.09 mmol) at 0 °C. The reaction mixture was warmed to RT and stirred for 1 h. Solvent was removed and the residue was purified by silica column (EtOAc/Hexanes=0/100 to 50/50 over 25 min) to afford L-9 as a white solid (2.44 g, 50%).

L-9 (440 mg, 0.450 mmol) and DTT (174 mg, 1.125 mmol) were dissolved in THF/water=20/1 and stirred for overnight. Solvent was removed and the residue was purified by silica column to afford thiol L-10 (300 mg, 68%) as a white solid.

R-3 (3.00 mg, 0.423 µmol) and L-10 (2.071 mg, 4.23 µmοl) were dissolved in THF/pH=6.8 Tris buffer= 10/1 600 µL and stirred for 3 h. The reaction mixture was purified by C₄ RP HPLC with TEAA as additive. The collected fractions was dialyzed 3 times against 3k membrane and lyophilized to give a white solid R-23 (0.61 mg, 19%, >95% purity).

Equal molar amount of guide strand G1 was mixed with compound R-23 to produce the corresponding double strand duplex C7-1. The duplex integrity was checked by CE analysis and the conjugate was submitted for biological evaluations.

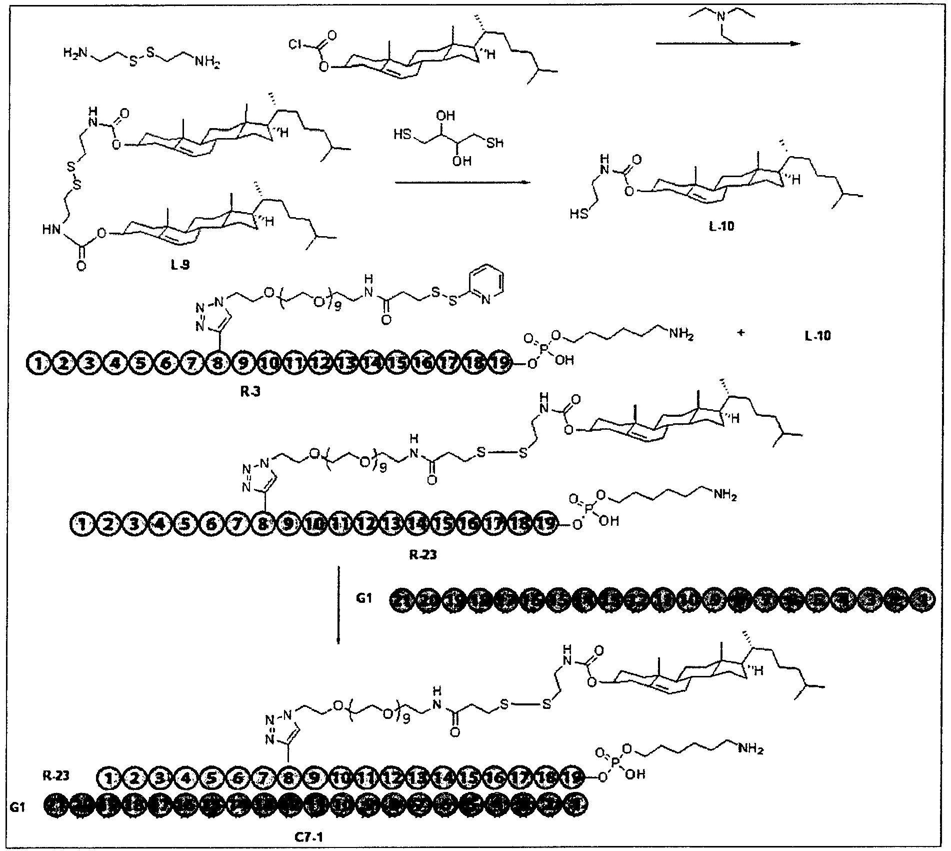

### EXAMPLE 8

A solution of R-2 (5.00 mg, 0.705 µmol) in 0.3 mL pH=8 Tris buffer was cooled to 0°C and treated with a solution of L-1 1 (2.76 mg, 4.93 µmol) in 0.3 mL MeCN. The resulting solution was stirred at room temperature for 0.5 h. The crude reaction was diluted with 18 mL water centrifugally dialyzed four times against water using a MW 3K dialysis membrane. The dialyte was lyophilized to provide the desired product R-24 as a fluffy white amorphous powder, measured mass = 7540.

A solution of R-24 (1.0 mg, 0.133 µmol) in 400 µL formamide/pH=6.8 Tris buffer=3/1 was treated with a solution of SEQ ID NO: 19 (2.164 mg, 0.530 µmol) in 400 µL formamide/pH=6.8 Tris buffer=3/1 and the resulting solution stirred at room temperature for 1.0 h. The crude reaction was purified by preparatory anion exchange chromatography on a Gilson apparatus using a 6 mL ResourceQ column and a 25-70% A over B linear gradient (A = 20 mM Tris-HCl, 50% formamide, pH=7.4; B = 20 mM Tris·HCl, 400 mM NaClO₄, 50% formamide, pH=7.4). Product peak was diluted with water, and was centrifugally dialyzed four times against water using a MW 10K dialysis membrane. The dialyte was lyophilized to provide 0.32 mg of the desired conjugate R-25 as a fluffy white amorphous powder.

Equal molar amount of guide strand G1 was mixed with compound R-25 to produce the corresponding double strand duplex C8-1. The duplex integrity was checked by CE analysis and the conjugate was submitted for biological evaluations.

Following the analogous procedures, RNA disulfide conjugate C8-2 to C8-6 were prepared and submitted for biological evaluations.

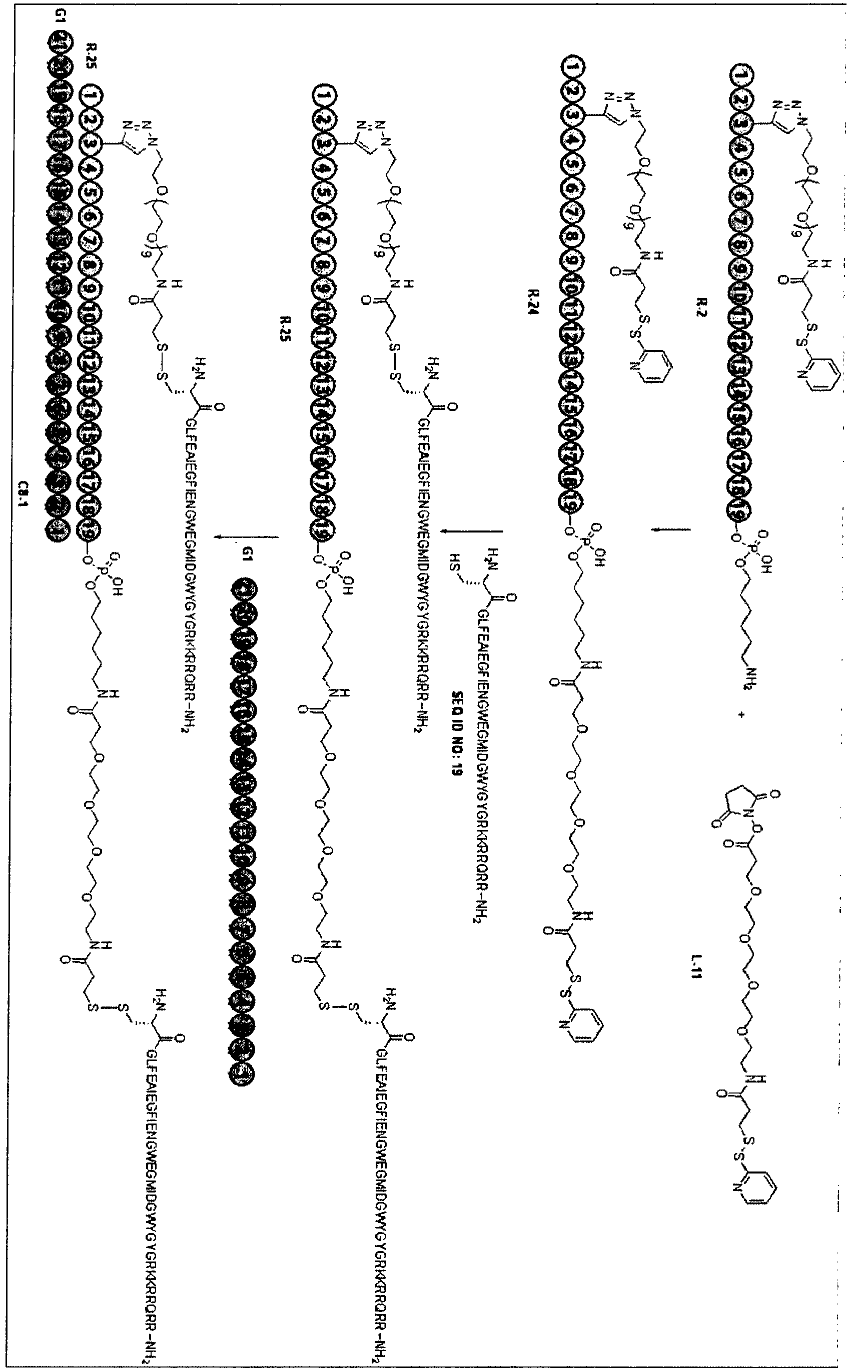

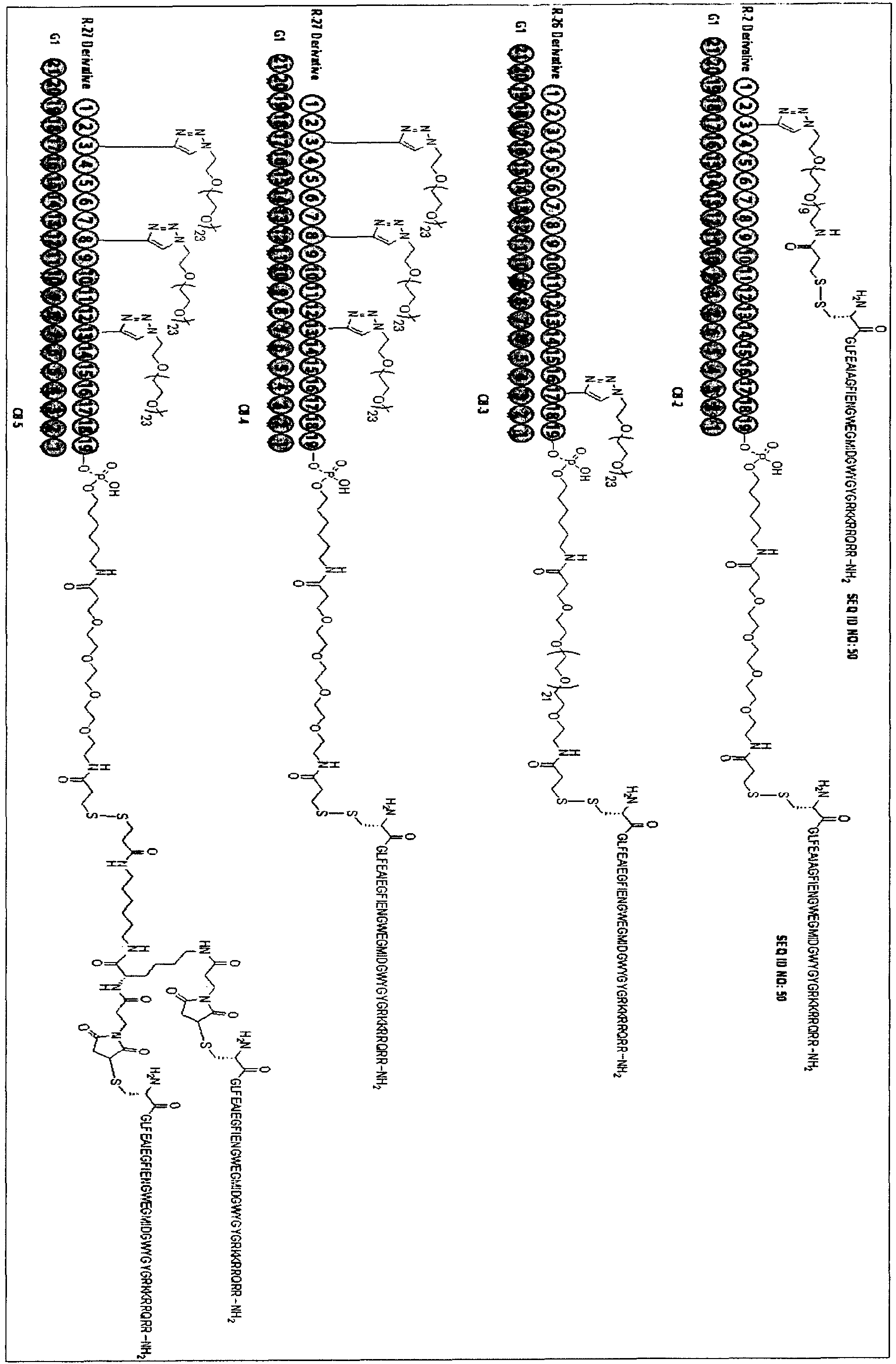

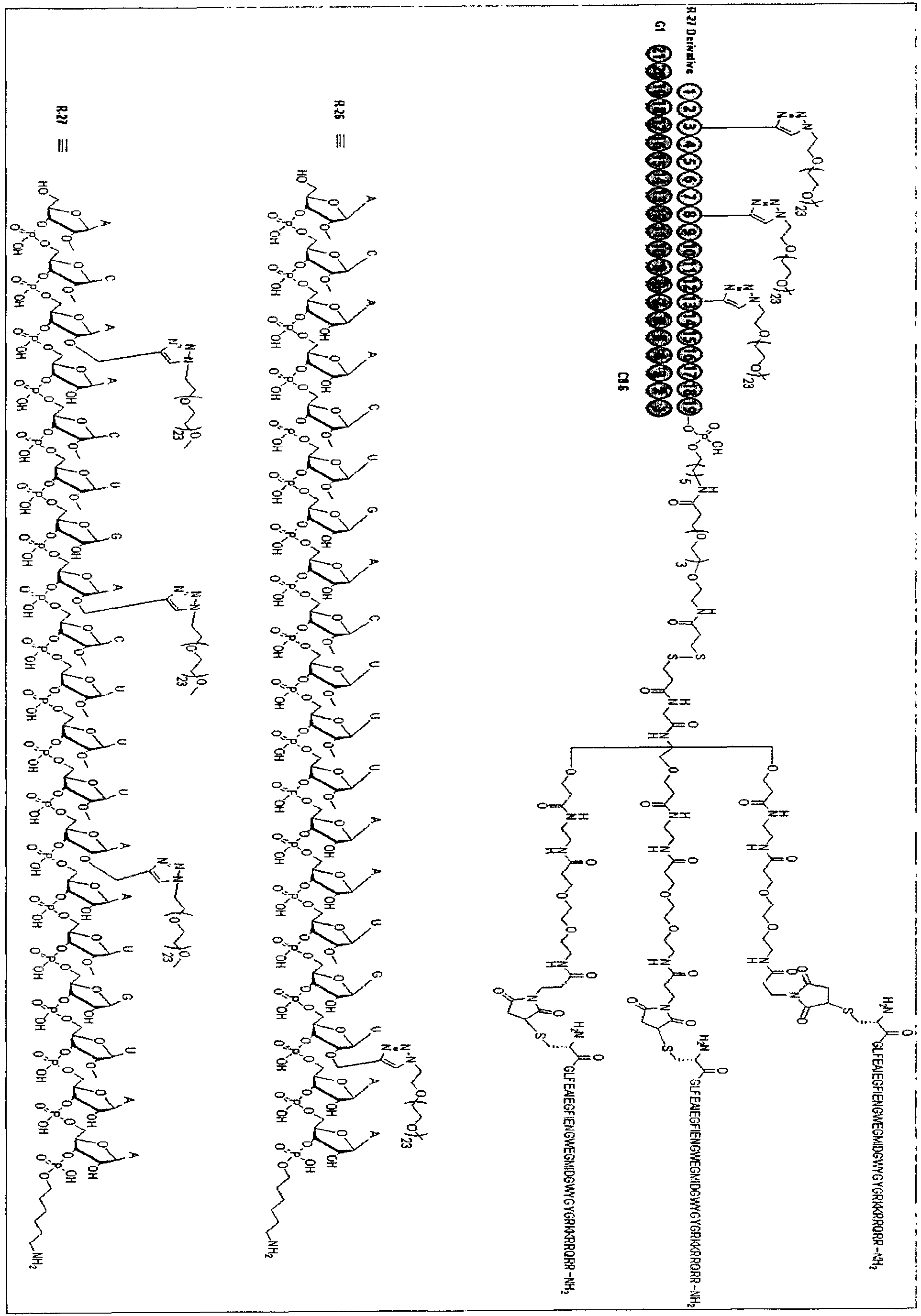

### EXAMPLE 9

Crude, oligonucleotide G3 50 mg was treated with azido-peg9-SPDP L-3 (38.6 mg, 0.053 mmol) and CuBr·Me₂S (2.74 mg, 0.013 mmol) in 3 mL of DMA/Water=3/1. The resulting reaction mixture was stirred for 48 h at room temperature followed by addition of 2.0 mL of 40% NH₄F/water=1/1. The biphase mixture was stirred at 65 °C for 1 h, then purified by C₁₈ cartridges to give a crude white solid G4.

Following the analogous procedures, RNA disulfides G5 and G6 were prepared respectively.

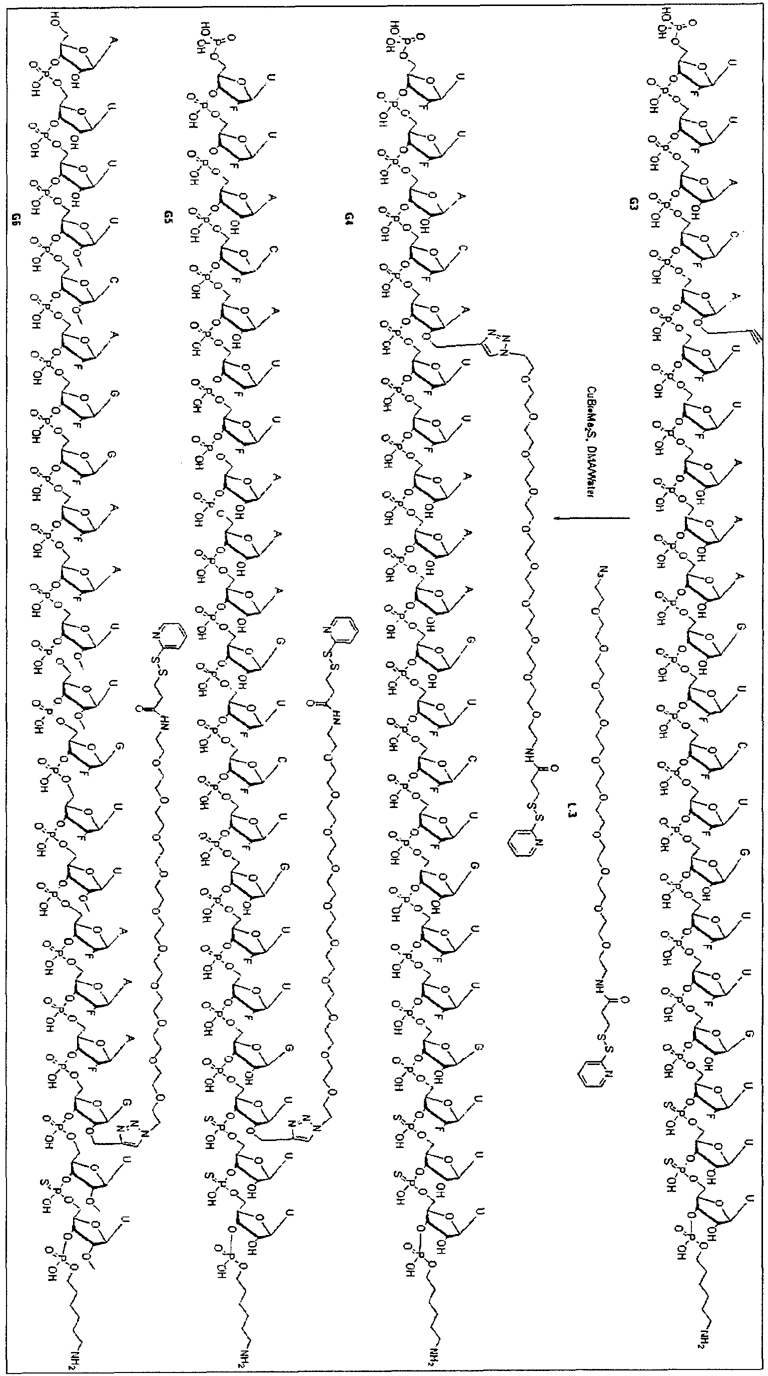

### EXAMPLE 10

Compound G4 (3.00 mg, 0.391 µmol) in 300 µL formamide/pH=6.8 Tris buffer=3/1 was treated with peptide SEQ ID NO: 19 (3.19 mg, 0.782 µmοl) in 300 µL of the same buffer and the resulting reaction mixture was stirred for 0.5 h. The reaction was diluted by addition of formamide/pH=6.8 Tris buffer=3/1 to a total volume of 2.5 mL and purified by strong anion exchange chromatography on a Resource Q column (25-75% B in A, A: formamide/H₂O=1/1, 20 mmol Tris·HCl, pH=7.4, B: formamide/H₂O=1/1, 20 mmol Tris·HCl, 400 mmol NaClO₄, pH=7.4). Combined product fractions were diluted with water, and centrifugally dialyzed 4 times against water with MW 10,000 cutoff membrane. The dialyte was lyophilized to provide G7 3.5 mg as a white solid, mass = 11640.

Equal molar amount of guide strand G7 was mixed with passenger strand R-28 to produce the corresponding double strand duplex C10-1. The duplex integrity was checked by CE analysis and the conjugate was submitted for biological evaluations.

Following the analogous procedures, RNA disulfide conjugates C10-2 to C10-8 were prepared respectively and submitted for biological evaluations.

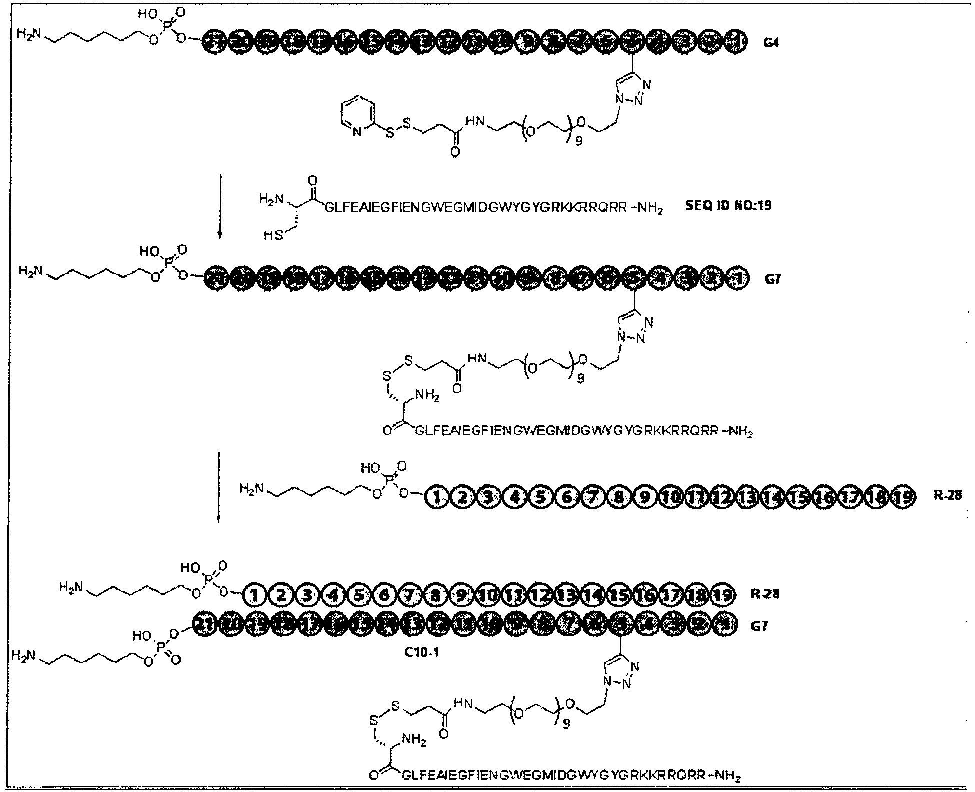

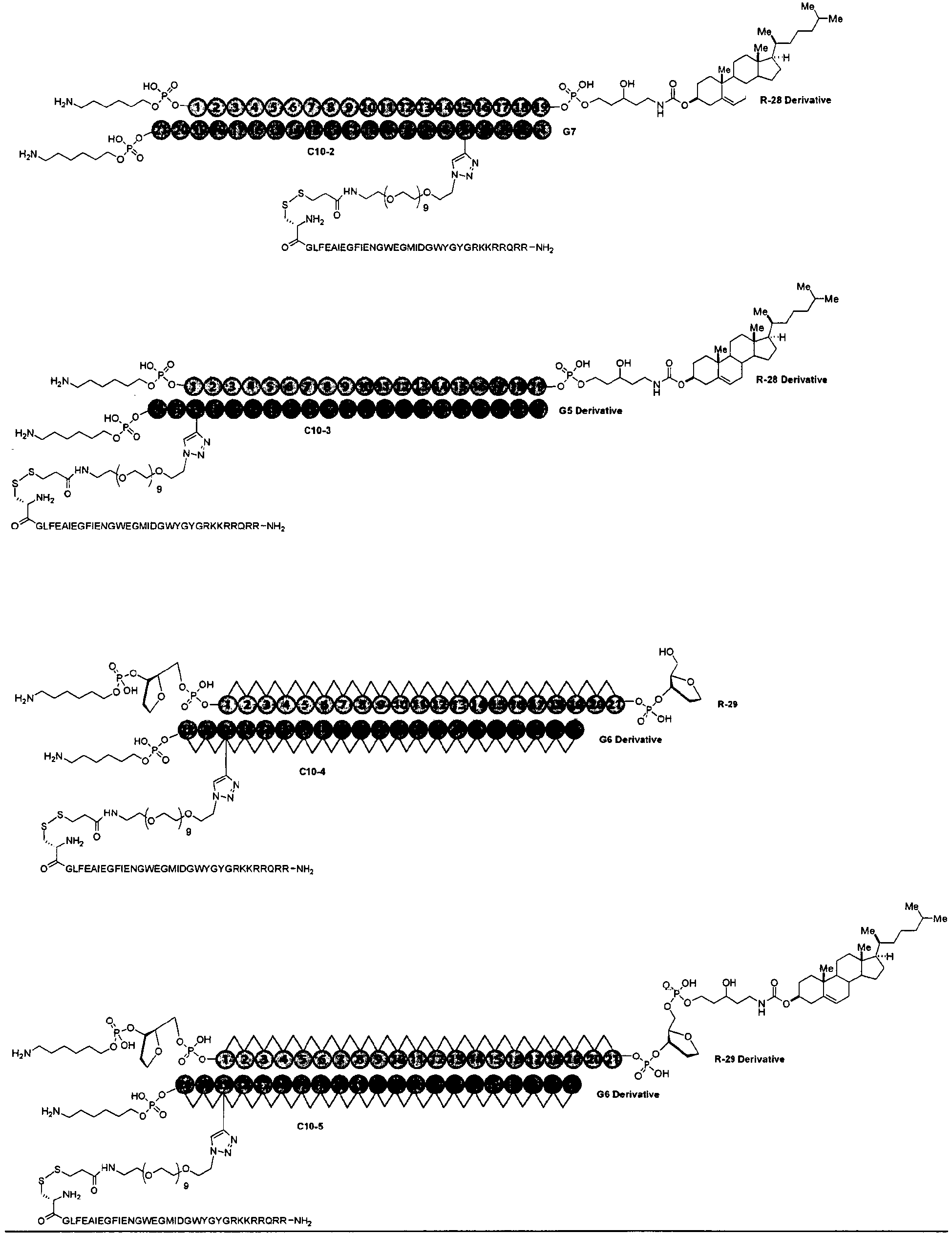

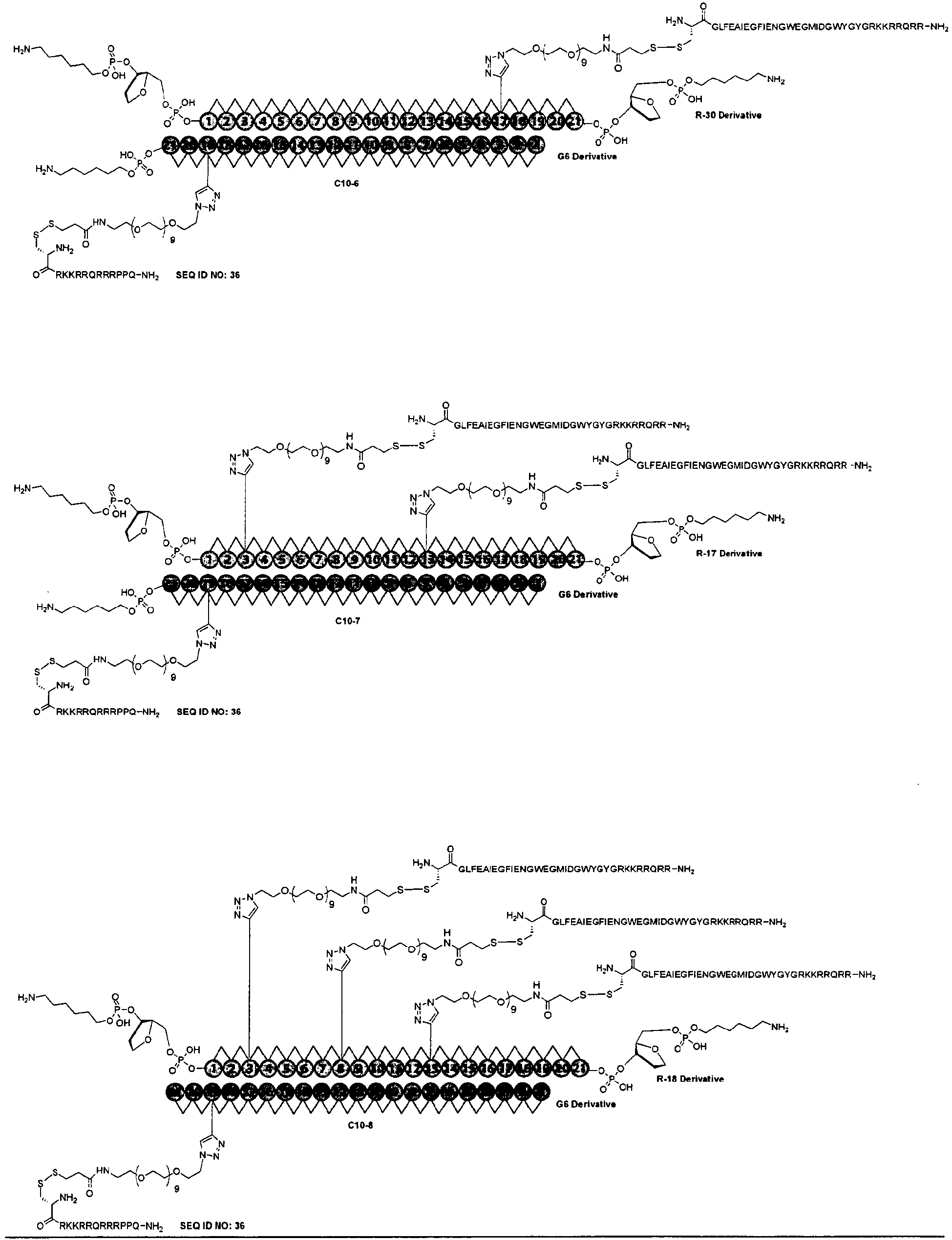

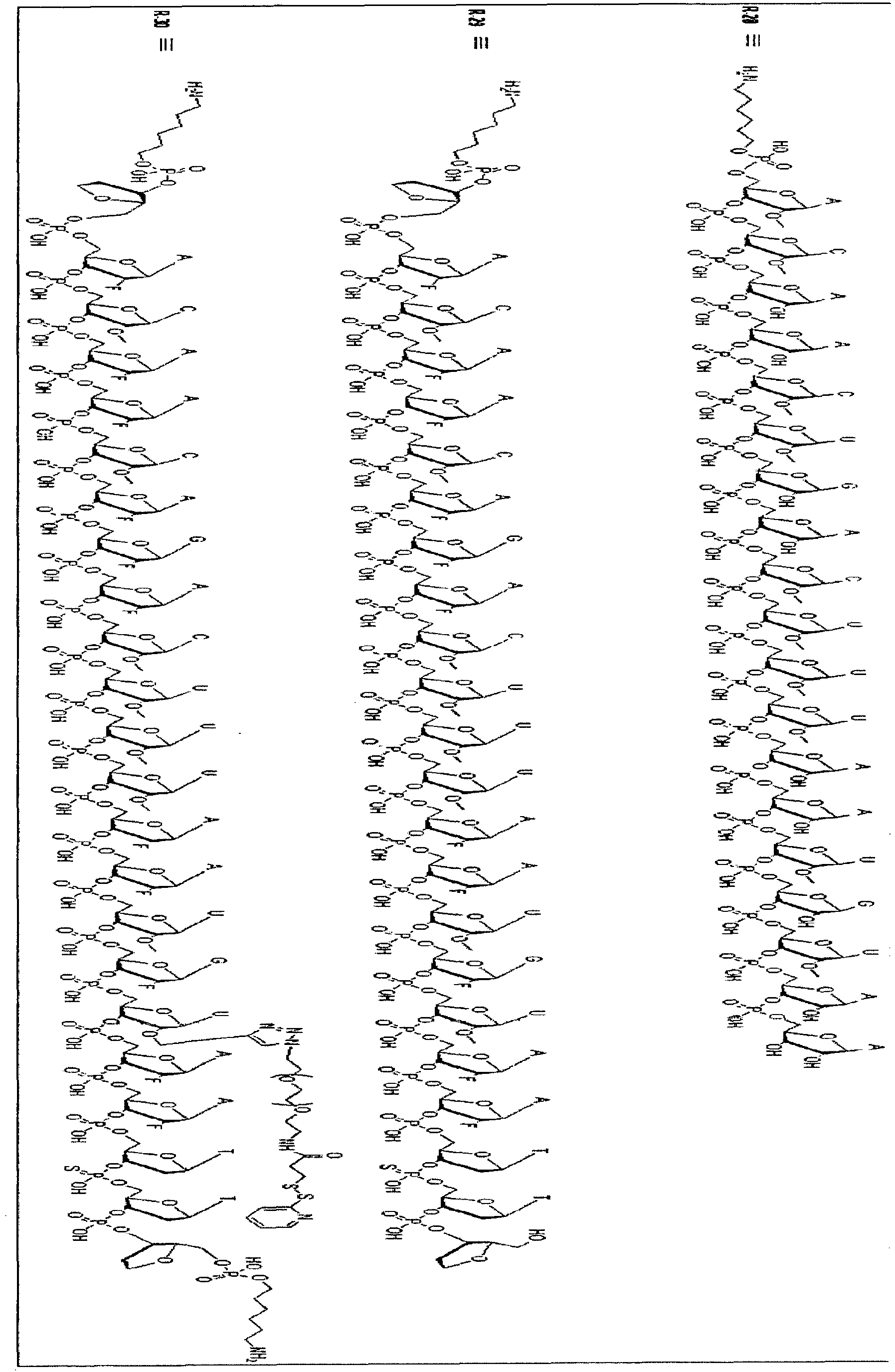

### ASSAYS

### siRNA Assay General Protocol

The siRNAs described herein were designed to target ubiquitously expressesd gene SSB (Sjogren syndrome antigen B; NM_009278.4). The sequence of the siRNA used is homologus in human, mouse and rat transcripts. To test the silencing activity of siRNA conjugates, HeLa (Human cervical cancer cell line) cells were plated in media (DMEM) supplemented with 10% fetal calf serum (FCS) and allowed to culture overnight (37°C, 5%CO₂). On next day, the media was replaced with serum free media containing the siRNA conjugates at concentrations ranging from 10-0.0015 µM and left on cells for total of 72 hrs (37°C, 5%CO₂). The SSB mRNA levels were analyzed using branched-DNA assay as per instructions by supplier (Panomics Quantigene 1.0 bDNA Kit # QG0002) or Luc assay. The cell viability was assessed using MTS assay (Promega cat# TB245) and all the data was normalized to levels from untreated cells.

The HeLa cells were treated with compounds indicated for 72 hrs in dose-dependednt manner and the levels of SSB mRNA were analyzed by b-DNA or Luc assay.

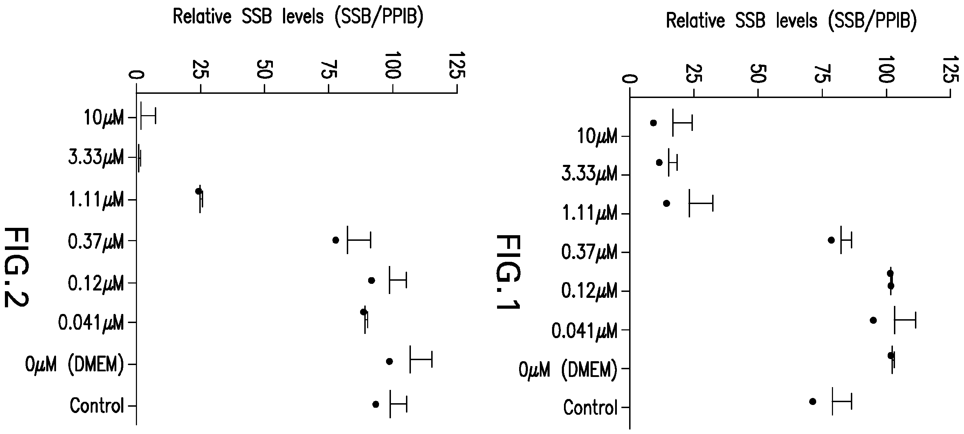

As shown in Fig. 1 SSB mRNA levels after HeLa cells were treated with compound C4-1 for 72 hrs (37°C, 5%CO₂).

As shown in Fig. 2 SSB mRNA levels after HeLa cells were treated with compound C4-5 for 72 hrs (37°C, 5%CO₂).

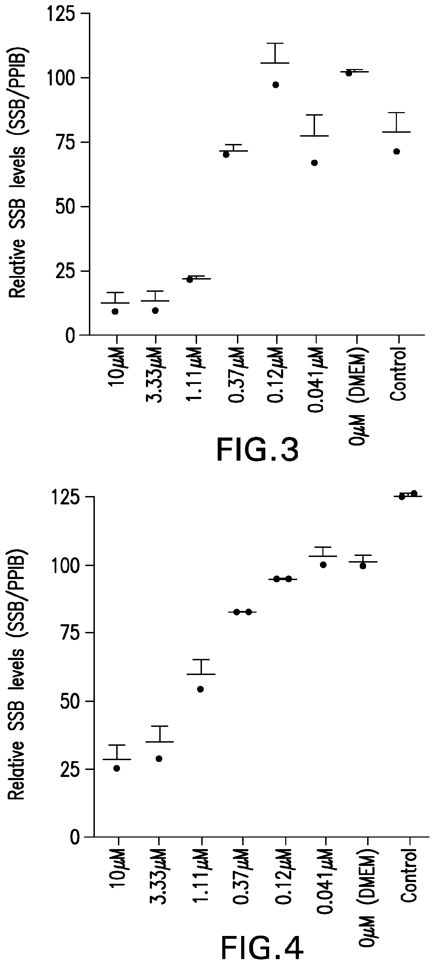

As shown in Fig. 3 SSB mRNA levels after HeLa cells were treated with compound C4-8 for 72 hrs (37°C, 5%CO₂).

As shown in Fig. 4 SSB mRNA levels after HeLa cells were treated with compound C4-10 for 72 hrs (37°C, 5%CO₂).

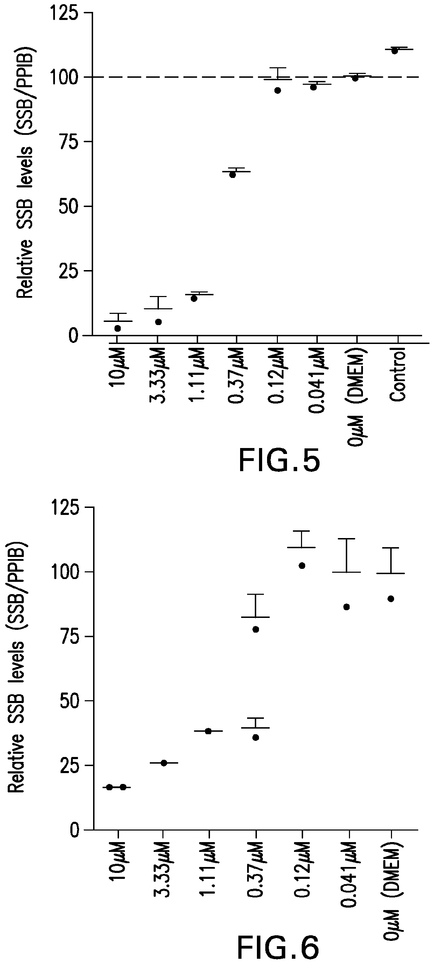

As shown in Fig. 5 SSB mRNA levels after HeLa cells were treated with compound C6-1 for 72 hrs (37°C, 5%CO₂).

As shown in Fig. 6 SSB mRNA levels after HeLa cells were treated with compound C6-2 for 72 hrs (37°C, 5%CO₂).

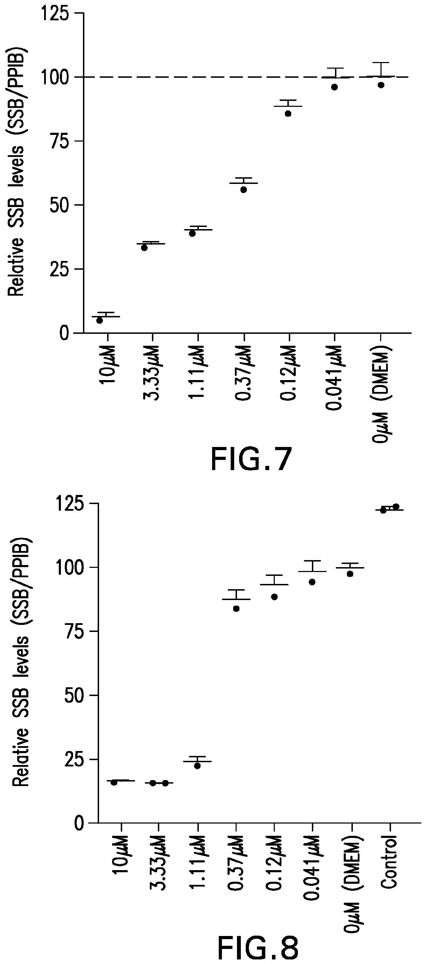

As shown in Fig. 7 SSB mRNA levels after HeLa cells were treated with compound C7-1 for 72 hrs (37°C, 5%CO₂).

As shown in Fig. 8 SSB mRNA levels after HeLa cells were treated with compound C8-1 for 72 hrs (37°C, 5%CO₂).

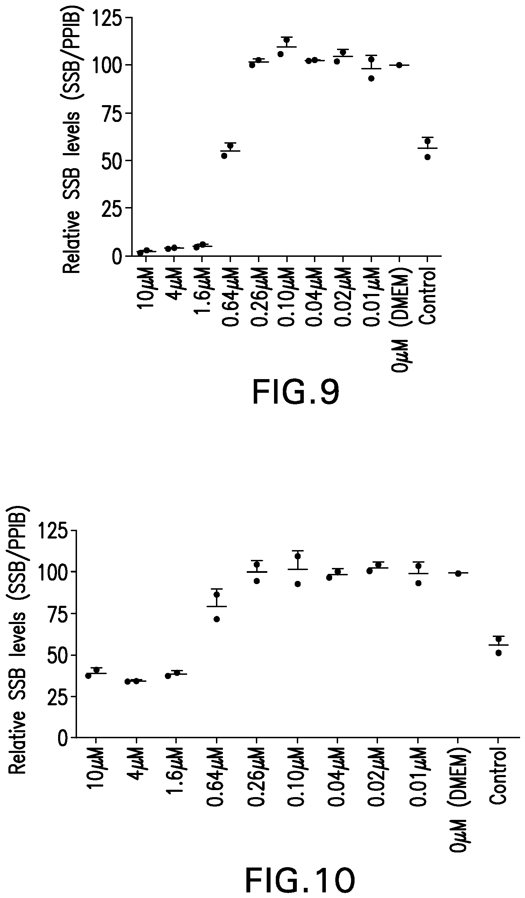

As shown in Fig. 9 SSB mRNA levels after HeLa cells were treated with compound C10-7 for 72 hrs (37°C, 5%CO₂).

As shown in Fig. 10 SSB mRNA levels after HeLa cells were treated with compound C10-8 for 72 hrs (37°C, 5%CO₂).

### In Vivo siRNA Assay General Protocol

All procedures involving animals were performed in accordance with the ARVO Statement for the Use of Animals in Ophthalmic and Vision Research and were approved by the Institutional Animal Care and Use Committee (IACUC) of Merck Research Laboratories, West Point, PA. Male Brown Norway rats (6-8 weeks) were purchased from Charles River Laboratories. siRNAs were prepared aseptically to minimize the risk of infection. For intravitreal dosing, rats were anesthetized with ketamine/xylazine (40-90/5-10 mg/kg, IM), and 1% proparacaine hydrochloride (1-2 drops) was applied to the eye as topical anesthetic. For intravitreal injection, pair of clean forceps was used to gently proctose and hold in place the eye, and a 30G sharp-needled syringe was used to inject 5µL of test siRNA or control vehicle into the vitreous just posterior to the limbus. On the day of sacrifice, rats were euthanized with sodium pentobarbital (150-200 mg/kg, IP). Following enucleation, vitreous, retina, and RPE/choroid were dissected and frozen.

Eye exams were performed just prior to intravitreal injection and just prior to harvest.

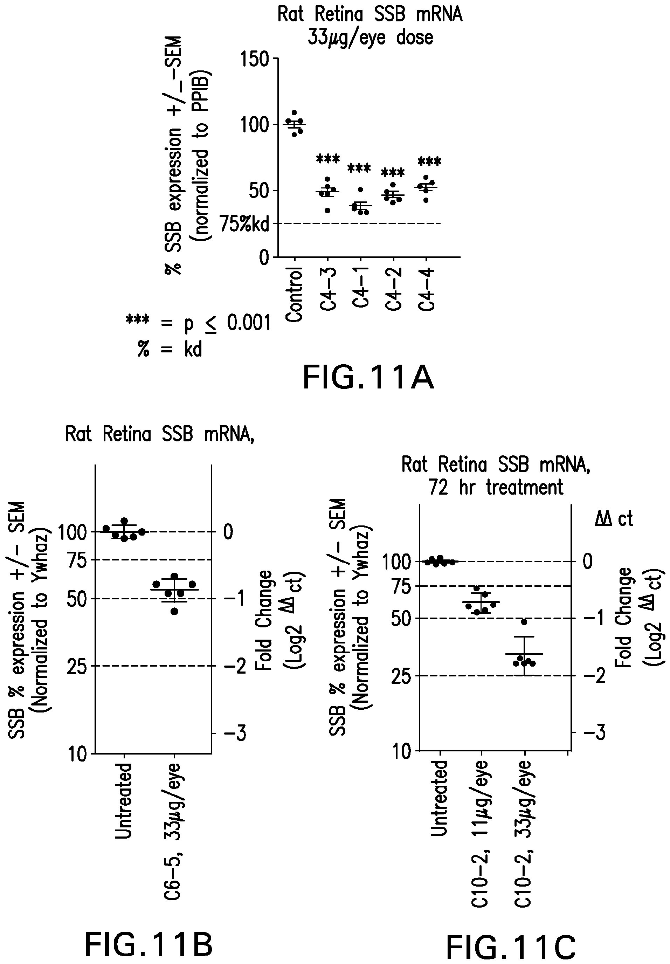

As shown in Figure 11A, SSB mRNA levels in rat retina for cojugates C4-1, C4-2, C4-3, C4-4.

As shown in Figure 11B, SSB mRNA levels in rat retina for cojugates C6-5.

As shown in Figure 11C, SSB mRNA levels in rat retina for cojugates C10-2 at 2 different dose.

## Claims

1. A modular composition comprising
1) an siRNA;
2) one or more linkers according to the following formula: 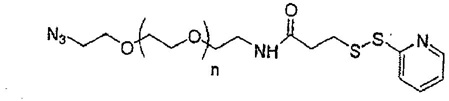 wherein n = 9,
wherein the linkers are attached to the guide strand of the siRNA at the 2'-position of the ribose rings excluding the terminal 3'- and/or 5'-positions of the guide strand, or to the passenger strand of the siRNA at the 2'-position of the ribose rings excluding the terminal 3'- and/or 5'-positions of the passenger strand; and
3) one or more peptides according to SEQ ID NO: 19, wherein the peptides are attached to the linkers.

2. The modular composition according to Claim 1 which further comprises cholesterol, wherein cholesterol is attached to the siRNA at the 2'-position of the ribose rings or the 3'-position of the siRNA.

3. The modular composition according to Claim 1 which further comprises cholesterol, wherein cholesterol is attached to the siRNA at the 2'-position of the ribose rings and/or the terminal 3'- and/or 5'-positions of the siRNA.

## Patentansprüche

1. Modulare Zusammensetzung, umfassend
1) eine siRNA,
2) einen oder mehrere Linker gemäß der folgenden Formel: 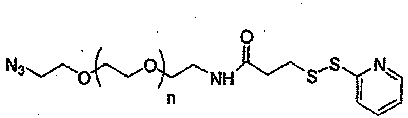 wobei n=9,
wobei die Linker an der 2'-Position der Riboseringe, unter Ausschluss der terminalen 3'- und/oder 5'-Positionen des Leitstrangs (*guide strand*), an den Leitstrang der siRNA, oder an der 2'-Position der Riboseringe, unter Ausschluss der terminalen 3'- und/oder 5'-Positionen des Begleitstrangs (*passenger strand*), an den Begleitstrang der siRNA angefügt sind und
3) ein oder mehrere Peptide gemäß SEQ ID NO: 19, wobei die Peptide an die Linker angefügt sind.

2. Modulare Zusammensetzung nach Anspruch 1, welche weiter Cholesterin umfasst, wobei das Cholesterin an der 2'-Position der Riboseringe oder der 3'-Position der siRNA an die siRNA angefügt ist.

3. Modulare Zusammensetzung nach Anspruch 1, welche weiter Cholesterin umfasst, wobei das Cholesterin an der 2'-Position der Riboseringe und/oder den terminalen 3'- und/oder 5'-Positionen der siRNA an die siRNA angefügt ist.

## Revendications

1. Composition modulaire comprenant
1) un pARNi ;
2) un ou plusieurs lieurs selon la formule suivante : 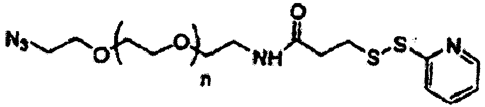 dans laquelle n = 9,
dans laquelle les lieurs sont fixés au brin guide du pARNi en position 2' des cycles de ribose en excluant les positions terminales 3' et/ou 5' du brin guide, ou au brin passager du pARNi en position 2' des cycles de ribose en excluant les positions terminales 3' et/ou 5' du brin passager ; et
3) un ou plusieurs peptides selon SEQ ID NO : 19, dans laquelle les peptides sont fixés aux lieurs.

2. Composition modulaire selon la revendication 1 qui comprend en outre du cholestérol, dans laquelle le cholestérol est fixé au pARNi en position 2' des cycles de ribose ou en position 3' du pARNi.

3. Composition modulaire selon la revendication 1 qui comprend en outre du cholestérol, dans laquelle le cholestérol est fixé au pARNi en position 2' des cycles de ribose et/ou en positions terminales 3' et/ou 5' du pARNi.
